(19) Europäisches Patentamt
European Patent Office
Office européen des brevets

(11) **EP 1 962 891 B1**

(12) **EUROPEAN PATENT SPECIFICATION**

(45) Date of publication and mention
of the grant of the patent:
**16.02.2011 Bulletin 2011/07**

(51) Int Cl.:
*A61K 39/00* (2006.01)  *C12N 15/62* (2006.01)
*A61P 35/00* (2006.01)

(21) Application number: **06832315.3**

(22) Date of filing: **19.12.2006**

(86) International application number:
**PCT/IN2006/000497**

(87) International publication number:
**WO 2007/072504 (28.06.2007 Gazette 2007/26)**

(54) **DNA VACCINE FOR CANCER THERAPY**

DNA-VAKZINE FÜR DIE KREBSTHERAPIE

VACCIN À ADN POUR THÉRAPIE ANTICANCÉREUSE

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **19.12.2005 IN DE33932005**

(43) Date of publication of application:
**03.09.2008 Bulletin 2008/36**

(73) Proprietor: **Fresenius Kabi Oncology Limited New Delhi 110 066 (IN)**

(72) Inventors:
• **MAITHAL, Kapil**
  **Ghaziabad 201 010,**
  **Uttar Pradesh (IN)**
• **SENGUPTA, Paromita**
  **Ghaziabad 201 010,**
  **Uttar Pradesh (IN)**
• **MUKHERJEE, Rama**
  **Ghaziabad 201 010,**
  **Uttar Pradesh (IN)**

(74) Representative: **Dzieglewska, Hanna Eva**
**Dehns**
**St Bride's House**
**10 Salisbury Square**
**London**
**EC4Y 8JD (GB)**

(56) References cited:
• **TELLAM JUDY ET AL: "Proteasomal targeting of a viral oncogene abrogates oncogenic phenotype and enhances immunogenicity."** BLOOD, vol. 102, no. 13, 15 December 2003 (2003-12-15), pages 4535-4540, XP002427942 ISSN: 0006-4971 cited in the application
• **KOECHLING J ET AL: "OPTIMIZATION OF DNA VACCINE STRATEGIES PROTECTS MICE AGAINST SYNGENEIC PH+ ALL"** BLOOD, W.B.SAUNDERS COMPANY, ORLANDO, FL, US, vol. 102, no. 11, 9 December 2003 (2003-12-09), page 746A,ABSTRNO2751, XP008037116 ISSN: 0006-4971
• **DENG HONYING ET AL: "A modified DNA vaccine to p53 induces protective immunity to challenge with a chemically induced sarcoma cell line"** CELLULAR IMMUNOLOGY, vol. 215, no. 1, January 2002 (2002-01), pages 20-31, XP002427943 ISSN: 0008-8749
• **ANDERSSON H A ET AL: "Maximizing Antigen Targeting to the Proteasome for Gene-Based Vaccines"** MOLECULAR THERAPY, ACADEMIC PRESS, SAN DIEGO, CA,, US, vol. 10, no. 3, September 2004 (2004-09), pages 432-446, XP004660584 ISSN: 1525-0016 cited in the application
• **REUBI JEAN CLAUDE ET AL: "Vasoactive intestinal peptide/pituitary adenylate cyclase-activating peptide receptor subtypes in human tumors and their tissues of origin"** CANCER RESEARCH, vol. 60, no. 11, 1 June 2000 (2000-06-01), pages 3105-3112, XP002427944 ISSN: 0008-5472 cited in the application

## Description

The present invention relates to a synthetic fusion gene comprising a sequence composed of ubiquitin gene (Ub) fused to a gene sequence encoding the extracellular domain of VPAC1 that is overexpressed in different types of cancer cells, useful as a therapeutic and prophylactic agent for treatment of cancers of various origins, especially useful as a DNA vaccine for cancer therapy.

In particular, the present invention relates to a synthetic fusion gene comprising a sequence composed of modified (mutated) ubiquitin gene fused to a suitably modified gene sequence of the extracellular domain of VPAC1.

## BACKGROUND OF THE INVENTION

The hallmark of a tumor vaccine is its ability to deliver specific antigen to the immune system and to induce immunity against weak tumor antigens, which have already successfully evaded the first line of immune defense due to immunological lapse.

The primary reason for the immunological lapse in cancer cells is the presence of "self antigens" in cells, which provide an easy mode for these cells to escape the immunological attack. Furthermore, the cancer cells have also evolved number of ways to evade the immune system like:

a) Dysfunction of the immune system which may arise from defect in antigen presentation (loss of MHC class I genes, TAP protein defects etc); T-cell defects due to depletion of arginine leading to T cell anergy and dendritic cell defects or depletion (by over expression of growth factors and certain cytokines like IL-10 and also nitric oxide and hydrogen peroxide which causes DCs to undergo cell death).

b) Immune suppression by increasing the levels of Cyclooxygenase-2 (COX-2), an enzyme produced by lung cancer cells, which causes immunosuppression and allows metastasis of cancer.

c) Cell deletion by some tumors, which actively kill developing T cells specific for the tumor antigen, which could potentially mount an anti-tumor response thus evading the immune challenge.

d) Immune ignorance where although T cells show an immune response against a reactive tumor antigen in vitro but is absent in vivo. Immune ignorance means that the T cells are unable to recognize the tumor antigen.

In spite of these hurdles, in recent years there has been a quantum leap in the area of cancer immunotherapeutics and they are on the threshold of taking their place alongside the more traditional cancer treatment modalities of surgery, radiation therapy and chemotherapy [Curr Opin Mol Ther, 2001, 3(1), 77-84].

In recent times there has been a great impetus in the area of tumor immunology and many different types of candidate vaccines are being tested for cancer treatment.

Cancer vaccines are categorized into (a) Tumor antigen based vaccines (b) Synthetic peptide/ protein vaccines (c) Carbohydrate based vaccines (d) Cell-based vaccines (e) Antibody based vaccines (f) DNA based Vaccines.

(a) Tumor antigen based vaccines

Tumor specific antigens (TSA) are unique to tumor cells and are absent in normal cells. TSA are present in tumors, induced by infectious agents (e.g. EBNA-1 antigen from Epstein Barr virus-induced Burkitt's lymphoma) and mutated genes found only in tumor cells (e.g. mutated caspase-8 enzyme found in head and neck cancer, which is different from the normal caspase-8).

Tumor associated antigens (TAA) are similar to regular cells but are either modified or produced in greater quantities. Some TAA are limited to a specific tumor type (e.g. melanocyte differentiation antigens, MAGE antigens, are limited to melanoma and some normal tissue), while other TAA are found in several different tumors (e.g. cancer testis antigen are found in normal testis and a variety of cancers such as prostate carcinoma, breast carcinoma, and lung carcinoma).

These TAA or TSA can be a peptide/protein antigen or a carbohydrate antigen. The peptide/protein antigen can be administered to the patient either as a DNA vaccine or a synthetic peptide/protein vaccine.

(b) Synthetic peptide/ protein vaccines

The methods of identification of CD8+ and CD4+ epitomes either by use of epitope prediction algorithms or use of transgenic mice has made the use of defined synthetic peptides more attractive. The possibility to synthesize long peptides and introduce multiple epitopes (CD4 or CD8) from single or multiple antigens makes peptide a viable alternative to whole proteins.

As an alternative to single or totally synthetic peptide constructs or polymers, polytopes are being used and are generated by genetic engineering methods [Cancer Res, 2002, 62, 5807-5812]. Further, there has been a tremendous amount of work in the development of delivery system of these immunogens and to activate dendritic cells (DCs) with the use of antibodies, cytokines and carbohydrates as some easy modes of delivery [Curr Med Chem.,

2006, 13(14), 1591-607]. Adjuvants are also added to peptide vaccines to stimulate the immune system.

One of the major disadvantages of peptide vaccines is the possibility of raising an irrelevant peptide- specific response. For instance, it was reported that patients immunized with her2 peptide developed her2- specific T- cell responses; however the reactive T cells failed to recognize her2+ tumor cells [Cancer Res., 1998, 58, 4902- 4908]. This suggests that the peptide may not be processed naturally by the tumor cell. This disadvantage is of particular concern when using peptides from patient specific mutated proteins, i.e., p53 and ras [The Oncologists; 2002, suppl3, 20-33].

(c) Carbohydrate based vaccines:

Individual tumor cells and early metastases are eliminated by inducing an antibody response against cell surface carbohydrate antigens [Scand J Immunol., 2005, 61, 391-397]. Thompsen-Freidenreich antigen, a small molecule found in carcinomas but not in normal cells, is expressed in early stages of cancer transformation. The use of this tumor antigen is an attractive option since it is shared by many types of cancers. Zhan et al. also demonstrated 28 different carbohydrates and glycoprotein antigens abundantly expressed on the surface of prostrate cancer specimens and cell lines. These included the glycolipid antigens like Lewis Y, Globo H, and GM2 [Clin. Cancer Res., 1998, 4, 295- 302]. Researchers focus on vaccines directed against these self-antigens as a means of breaking immunological tolerance.

One of the major drawbacks of carbohydrate vaccine is the poor immunogenicity associated with it [Semin Oncol., 2003, 30, 659- 666]. GM2 is one of the most immunogenic gangliosides that has been the target of several vaccination clinical trials for patients with melanoma. Vaccination with GM2/BCG generate high- titer antibodies, specific to the immunizing antigens, but there was no evidence of antitumor effect. Furthermore, carbohydrate based drugs display unfavorable pharmacokinetics and often have poor metabolic stability and poor oral adsorption [Mini Rev Med Chem., 2003, 3, 633- 649].

(d) Cell-based immunotherapy:

These are cancer vaccines, which involve administration of an entire cell population into the patient.

    (i) Tumor cell-based vaccine: Whole cells (containing tumor antigens), taken either from the patient (autologous) or from a different patient (allogeneic), are introduced to stimulate the immune system to recognize the existing tumor and mount a response against it.

    Some of the earliest attempts at cancer vaccination have used allogeneic melanoma cells cultivated in tissue culture as vaccines. The assumption was that in the whole cell milieu there would be some relevant tumor-specific antigens that would be recognized by the immune system. Multiple variations of allogeneic cell vaccines have been developed using whole irradiated cells [Int J Cancer, 1983, 31, 567- 575] cell lysates [Semin Sur. Oncol., 1989, 5, 420- 425] and shed antigens isolated from tissue culture supernatants [Cancer Res., 1992, 52, 5948- 5953].

    Another approach to cancer vaccine have been the use of autologous tumor cells, which are more likely to contain antigens of specific immunological importance for the individual patient, including any unique antigens resulting from mutations. The absence of irrelevant allogeneic antigens makes immunological monitoring more straightforward [Semin Oncol., 2003, 30(5), 659-666] But this approach requires a relatively large amount of tumor tissue to be available from each patient for developing customized vaccines, which restricts the 'eligible' patient population to only those, which have a relatively higher burden of the disease. Different strategies have been approached to increase the immunogenicity of autologous tumor cells, which includes conjugation of haptendinitrofluoro benzene (DNFB) to proteins on autologous cells [Cancer Res., 1991, 51, 2731- 2734] introduction of genes encoding different cytokines [Cancer Res., 1999, 59, 5160- 5168] and costimulatory molecules like CpG nucleotides etc.

    (ii) Dendritic cell-based vaccines: Dendritic cells (DCs) are bone- marrow- derived monocytic cells with an exceptional capacity for antigen presentation. Lack of efficient tumor antigen presentation in DCs in cancer patients has led to the use of DC-based vaccines. The tumor antigens are taken up by dendritic cells; they are processed and presented to the T cells along with the appropriate costimulatory signal. Once activated by the DCs the cytotoxic T cells recognize and destroy the tumor cells expressing the tumor antigen. Techniques have been developed for the *ex-vivo* expansion and loading of DCs with peptides of interest, followed by reinfusion into patients [Immunol lett., 2000, 74, 87-93; Prostate, 1999, 39, 54-59; Brit. Med.J., 2001, 323, 1289-1293]. Loading of DCs with tumor RNA has also been investigated as a vaccination strategy. Loading with RNA allows for endogenous production and processing of antigens [J Clin Invest, 2002, 109, 409-417]. But the *ex-vivo* expansion of DCs is a labor-intensive approach. Hence, *in vivo* strategies have been exploited and explored to develop means of recruiting the DCs to the desired sites. One approach is the use of GM-CSF, either as a protein or as a DNA formulation, resulting in the infiltration of the DCs at the site of application [Cytokines Cell Mol Ther., 1999, 5,139- 144]. After some days, the same site is used to administer the vaccine (peptide, DNA, virus, etc). The hypothesis is that the locally concentrated DCs will present antigens efficiently and induce an immune response to the antigen. Preclinical studies in animals have demonstrated that administration of murine GM-CSF gene results in recruitment of epidermal DCs. Simultaneously, GM-CSF DNA acts as an adjuvant for peptide immunization and potentiates the tumor immunity resulting from DNA immunization [Cytokines Cell Mol Ther, 1999, 5, 217-225].

In Dendritic- cell based vaccines the *ex-vivo* expansion and loading of DCs is a labor- intensive and expensive approach. Vaccine production involves harvesting autologous DC from cultured peripheral blood mononuclear cells in the presence of a cocktail of cytokines, *ex vivo* exposure of the DC to tumour antigens and return of pulsed DC to the patient to induce tumour immunity [Semin Oncol., 2003, 30, 659-666]

(iii) T cell-based vaccines: Development of techniques of culturing T cells, and development of vectors to introduce appropriate genes, into these cells has led to clinical trials using T cells [Brit J Cancer, 2005, 93, 1085-1091]. The type of T cell used in immunotherapy may enrich immune responses and augment anti-tumor effect. Based on murine models, there appear to be two distinct populations of CD4+ cells (Th1 and Th2) that can be distinguished by their cytokine profiles. Th1 cells secrete interferon y (IFN $\gamma$) and tumor necrosis factor $\alpha$(TNF $\alpha$) that mediate responses to infections and also tumor cells and Th2 cells secrete cytokines that help B cells to proliferate and differentiate [Annu Rev Immunol., 1997, 15, 297-322] Similarly, the CD8+ subset has analogous Th1 and Th2 cells and are designated as Tc1 and Tc2 respectively [Immunity, 1995, 2, 271-279]. So the desired T-cell population can be adoptively transferred into the patient and is known as allogeneic lymphocyte therapy. Alternately, the CTL (cytotoxic T lymphocyte) cells themselves can be modified in such a way that they recognize the tumor antigens and become activated. For this purpose a fusion protein consisting of an antibody and a T-cell receptor (TCR) is used. The antibody recognizes the appropriate tumor and the TCR activates the T cell to combat the tumor. These have been successful in treating ovarian tumors in mice [Brit. Med J., 2001, 323, 1289-1293].

(iv) Natural killer (NK) cell-based therapy: Similar to the T cell-based strategy NK cells can also be used. Natural killer (NK) cells are effectors of the innate immune system that play an important role in host response against viruses and tumor cells through the production of cytokines and direct cytolytic activity. With a better understanding of the biology of NK cells, NK cell receptors and the role of interleukin-15 has led to the development of new strategies using NK cells as immunotherapy for cancer treatment in the transplant and non-transplant settings [Expert Opin Biol Ther., 2006, 6(10), 967-970]

Whole- cell based vaccine has major disadvantages as well, which includes (1) the actual amount of any TAA or TSA would be diluted by normal cellular components; (2) vast majority of human tumors, with the exception of hematopoietic malignancies, do not express costimulatory molecules and thus would not activate naïve T cells to levels capable of inducing therapeutic responses; and (3) one does not know, when using this approach, what are the immunogenic proteins or epitopes. Therefore, it is difficult to measure immune responses and to amplify those specific responses.

(e) Antibody based vaccine.

These are primarily an anti-idiotype vaccines. An anti-idiotypic antibody is an antibody against an individual structural determinant of variable region of other antibodies; therefore these antibodies bind to the tumor by recognizing the tumor antigen. The anti-idiotypic antibodies are also recognized by other antibodies, thus forming a network and enhancing the immune response. This triggers antibody-dependent cell cytotoxicity and T cell response, stimulating both humoral and cell-mediated immunity. The response is quite strong and long lasting hence obligating the need of any adjuvant [Handbook of Cancer Vaccines, 2004, 157-173].

(f) DNA based Vaccines:

Naked DNA immunization has emerged as an attractive approach for vaccine development [Vaccine, 1994,12, 1503- 1509] in which the inoculated plasmid DNA enters the cells and the encoded protein is expressed therein, ensuring access of the antigen to the major histocompatibility complex (MHC) class I antigen pathway [J Leukoc. Biol, 2000, 68,793- 806]. Furthermore, proteins released from transfected cells can interact with B-lymphocytes, inducing antibodies, and can be taken up by specialized antigen- presenting cells (APCs), allowing presentation by MHC class II. Thus, DNA immunization induces both arms of the immune response.

DNA vaccines are safer than live attenuated vaccines for administration to pregnant or immunocompromised individuals [Annu Rev. Immunol, 2000, 18, 927-974]. DNA vaccines can be manufactured in a relatively cost-effective manner and stored with relative ease, eliminating the need for refrigeration. The main challenge in these vaccines is to have an optimal expression of the gene and mode of delivery. Recently, DNA vaccines have emerged as an attractive approach for vaccine development. Many laboratories worldwide have made attempts to develop vaccines and immunotherapeutics based on plasmid DNA. These vaccines have been shown to induce strong immune responses against proteins from infectious agents such as malaria [Immunol. Cell Biol, 1997, 75, 376- 381; Immunol Cell Biol., 1997, 75, 370-375] tuberculosis [Immunol. Today, 1995, 16, 338-342 ; Immunol Cell Biol., 1997, 75, 364-369] rabies virus [Virology, 1994, 199, 132-140] hepatitis B virus [Vaccine, 1994, 12, 1503-1509] and HIV [Clin. Immuno., 1999, 90,100-107; DNA Cell Biol., 1993, 12, 799-805].

DNA vaccines have also been investigated for use in cancer. Vaccination with a DNA vector encoding a BCR-ABL$^{p185}$ fusion protein was able to prevent Ph$^{+}$ALL (Philadelphia chromosome positive acute lymphoblastic leukaemia) in mice injected with leukaemia cells. However, protection was reduced when the mice were vaccinated with a DNA vector encoding a BCR-ABL$^{p185}$-ubiquitin fusion protein [Blood, 2003, 102, 11, p.746 A]. Similarly the efficacy of a DNA vaccine encoding p53 in inducing protective immunity in mice challenged with cells from a sarcoma cell line that expresses high levels of mutated p53 has been investigated. Fusion of the p53 mutant protein to the signal sequence of the lysosome-associated membrane protein (LAMP) or ubiquitin did not improve the efficacy of the vaccine [Cellular Immunology, 2002, 215, 20-31].

However, major limitations of these DNA vaccines lies in their potency, because they do not have the intrinsic ability to amplify and spread *in vivo* as some replicating viral vaccine vectors do. Strategies that enhance DNA vaccine potency are explored for more effective cancer immunotherapy.

The present invention is a step forward in the direction of engineering of antigen to target specific cellular compartments that can enhance CTL response. The engineering of antigen that can enhance CTL response has been presented with use of growth factor receptors, which are overexpressed in cancer cells as the antigen of choice.

## OBJECTS OF THE INVENTION

An object of the present invention is to provide a synthetic fusion gene useful as a DNA vaccine for cancer therapy.

Another object of the invention is to provide a synthetic fusion gene useful as a DNA vaccine, which specifically targets cancer cells, over expressing desired receptors (antigen).

Yet another object of the invention is to provide a DNA vaccine, which will be preferably taken up by MHC class I pathway.

Still another object of the invention is to provide a DNA vaccine, which eliminates the target cancer cells by inducing cytotoxic T- lymphocyte (CTL) response.

Further object of the invention is to provide a DNA vaccine, useful as a therapeutic and prophylactic agent for treatment of cancers of various origins.

## SUMMARY OF THE INVENTION

In accordance with the above and other objects, the present inventors have designed and found that a synthetic gene comprising of sequences of ubiquitin gene (Ub) fused to the gene sequence of the extracellular domain of a growth factor receptor that are overexpressed in different types of cancer cells inhibits the growth of cancer cells as well as prevent the growth of cancer cells and hence useful both as therapeutic and prophylactic agents for cancer therapy.

Thus, one aspect of the present invention includes a synthetic fusion gene comprising a sequence composed of an ubiquitin gene fused to a gene sequence encoding the extracellular domain of vasoactive intestinal receptor type 1, which is over expressed in cancer of various origins.

A further aspect of the present invention includes a DNA vaccine comprising a sequence composed of an ubiquitin gene fused to a gene sequence encoding the extracellular domain of vasoactive intestinal receptor type 1, which is over expressed in cancer of various origins.

In particular, the present inventors have designed and found that a synthetic fusion gene comprising of mutated sequences of ubiquitin gene (Ub), wherein the C-terminus codon for glycine amino acid residue of the ubiquitin (gly76) has been substituted with codons for non-polar amino acids like valine to prevent cytosolic degradation of the fusion protein and to target the protein to the ubiquitin fusion degradation (UFD) pathway. The mutated sequences of ubiquitin gene (Ub) is fused to a suitably modified gene sequence of the extracellular domain of the VPAC1 growth factor receptor, in particular VPAC1 wherein a valine residue has been incorporated towards the N- terminus of the VPAC1 gene to enhance the targeting of the fusion protein to the UFD pathway and additional amino acids have been incorporated (in this case Asp, Ile) to generate a restriction enzyme site for proper linkage of the Ub and VPAC1 gene, inhibits the growth of cancer cells as well as prevent the growth of cancer cells and hence useful both as therapeutic and prophylactic agents for cancer therapy.

More specifically, the present invention is useful as a prophylactic agent especially in the form of a DNA vaccine for cancer therapy.

Therapeutic vaccines attempt to treat chronic infectious conditions by stimulating the immune system to more adequately defend the body in the situations when the patient is unable to develop an effective immune response. Therapeutic cancer vaccines aim to stop or retard the growth of existing tumors and also aim to prevent recurrence of cancer.

A prophylactic vaccine is a vaccine designed to prevent the growth of cancer. The vaccine may be administered prior to exposure to the pathogen, or after exposure to a pathogen but prior to the occurrence of disease. Prophylactic cancer vaccine could be given to an individual before cancer develops and could aim at stimulating the immune cells so that the tumor growth can be stopped or retarded on subsequent challenge.

The fusion gene of the invention activates the cell mediated immunity pathway and causes the differentiation of T- cells into CD8+ killer T- cells. The fusion of the antigen with ubiquitin causes enhanced translation and proteasomal targeting of the antigen, thus, inducing anti-tumor immunity mediated by CD8+ T cells. The vaccine will be most effective in situations where the biology of the antigen prevents efficient immune presentation or stimulation.

The constructed gene is used for cloning and expression in mammalian system. The DNA vaccine of the present invention can be used to treat diseases including various forms of cancers such as T- and B- cell lymphoproliferative diseases, ovarian cancer, breast cancer, pancreatic cancer, head and neck cancer, squamous cell carcinomas, melanoma, gastrointenstinal cancer, and non-small cell lung cancer. The vaccine can be used both for prophylactic and therapeutic purposes in a mammal.

The Ub-VPAC 1 fusion gene was constructed by recombinant DNA technology. Strategy for the construction of fusion gene is described in Figure-1. The nucleotide sequence and the corresponding amino acid sequence of the construct have been described in Figure-2. The Ub gene of 228bp and the VPAC1 gene of 348 bp were amplified from the cDNA of the colon carcinoma cell line HT-29 (Figure-3). The gene(s) were then ligated and amplified using PCR using specific primers, resulting in the construction of the Ub-VPAC1 fusion gene, with modifications as discussed above to give a final construct of 606 bp with expressible region of 588 bp (Figure-4). The ligated product was then inserted into a pcDNA3.1HisA expression vector under the control of the CMV promoter to yield an expression plasmid encoding Ub-VPAC1 protein.

Similarly, only the VPAC1 gene was ligated to the pcDNA3.1HisA expression vector to yield an expression plasmid encoding VPAC1 protein.

Expression of the VPAC1 and the Ub-VPAC1 gene was checked at the RNA level by RT-PCR. B16F10 (Murine melanoma) cells were transfected with the constructs- pVPAC1; pUbVPAC1 and the mock plasmid. 24 hours post-transfection, total RNA was isolated from the transfected cells, using Trizol reagent and treated with DNAse1 to rule out any possible contamination with the plasmid. RT- PCR was carried out to check for the expression of the gene(s). For checking the expression of VPAC1 gene, combination of primers were chosen in such a fashion [sense primer corresponding to vector sequence, (SEQ ID 6) and antisense primer corresponding to the VPAC1 gene sequence, (SEQ ID 4)] that only the VPAC1 encoded by the plasmid was amplified. This was essential as B16F10 cell line also has an intrinsic VPAC1 expression (Figure-5). Furthermore, respective control with untransfected cell line was also taken to rule out the possibility of any erroneous observations. The products were analyzed on agarose gel (Figure-6a). DNA band of 508 bp (corresponding to VPAC1 along with the additional vector region) was seen in the sample prepared from the transfected cells.

For confirming the expression of Ub-VPAC1 gene, a combination of primers were chosen such that the sense primer corresponds to the Ub sequence (SEQ ID1) and the antisense primer corresponds to the VPAC1 sequence (SEQ ID 4). A DNA band of 606 bp, corresponding to the fusion gene was seen on the agarose gel (Figure-6b). No band was detected for control RNA.

To evaluate the potential of the constructs as a candidate for good DNA vaccine, further studies were carried out using murine melanoma cell line B16F10. The effect of Ub conjugation on the processing and presentation of VPAC1 for CTL induction following DNA injection was evaluated against B16F10 cells at different Effector to Target cell ratios. Three different groups of C57BL/6J mice were immunized with plasmid DNA vectors encoding VPAC1, Ub-VPAC1, or the vector alone. Following immunization, the spleen cells were isolated from each group and CTL assay was carried out using the CytoTox96 non- radioactive cytotoxic assay kit. As shown in Figure-7, mice immunized with plasmid DNA encoding VPAC1 showed low levels of CTL activity. In contrast, an enhanced CTL response of ~40% at effector: target ratio of 50:1 was observed following immunization with an expression vector encoding ubiquitinated VPAC1. Very negligible amount of CTL activity was detected in mice immunized with vector alone. Furthermore, this cytotoxicity could be reduced by anti- CD8, but not by anti- CD4 *in vitro,* suggesting that the killing activity observed may result from MHC class 1 dependent CD8+ CTL activity (Figure-8).

To investigate the protective antitumor immunity, C57BL/6J mice were immunized by the intramuscular injection of plasmid (pVPAC1, pUb-VPAC1, vector alone) three times at an interval of 14- days each. And then challenged with B16F10 tumor cells. As shown in Figure-9, tumor grew progressively in mice immunized with pVPAC1 or vector alone. But there was an apparent reduction in tumor growth in pUb-VPAC1 immunized mice. At the 22$^{nd}$ day post- tumor injection, there was ~ 1.6 fold difference in the tumor volume of pVRAC1 and pUb-VPAC1 immunized mice.

Similarly, the therapeutic effects of this DNA vaccine were checked against melanoma implanted before vaccination. As shown in Figure-10, immunization of mice with pUb-VPAC1 resulted in marked suppression of tumor growth compared with mice immunized with vector or pVPAC1 plasmid. There was no effect of immunization of pVPAC1 on tumor regression. At the 21$^{st}$ day post- tumor injection, there was ~ 2.3 fold difference in the tumor volume of pVPAC1 and pUb-VPAC1 immunized mice. These results strongly indicate that immunization with plasmid DNA encoding the Ub-VPAC 1 gene is effective even after development of the tumor.

Comparing the tumor growth data of the two studies (Figure-9 and Figure-10), it is seen that although the prophylactic protocol is effective in preventing subsequent tumor challenge, inhibition of established tumor growth seems more pronounced. The mice immunized with these vaccines were in particular investigated for the potential long- term toxicity. No adverse consequences were indicated in gross measures such as weight loss, ruffling of fur, and life- span. These findings confirm both the therapeutic and prophylactic potential of ubiquitinylated VPAC1 vaccine, thus enhancing of its potential clinical effectiveness against different types of cancers.

## BRIEF DESCRIPTION OF THE DRAWINGS

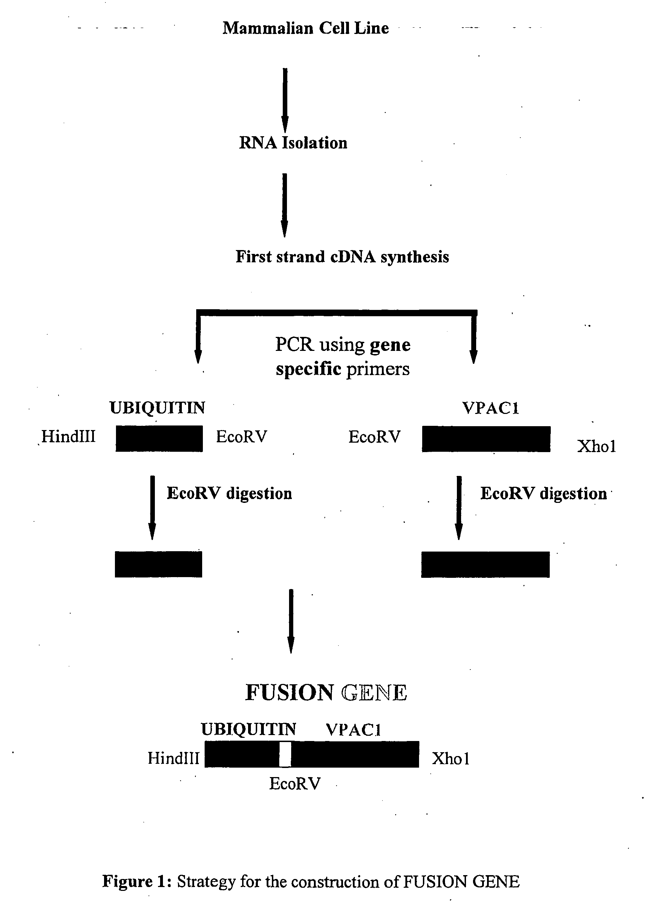

**Figure 1:**  summarizes the strategy for construction of fusion gene.

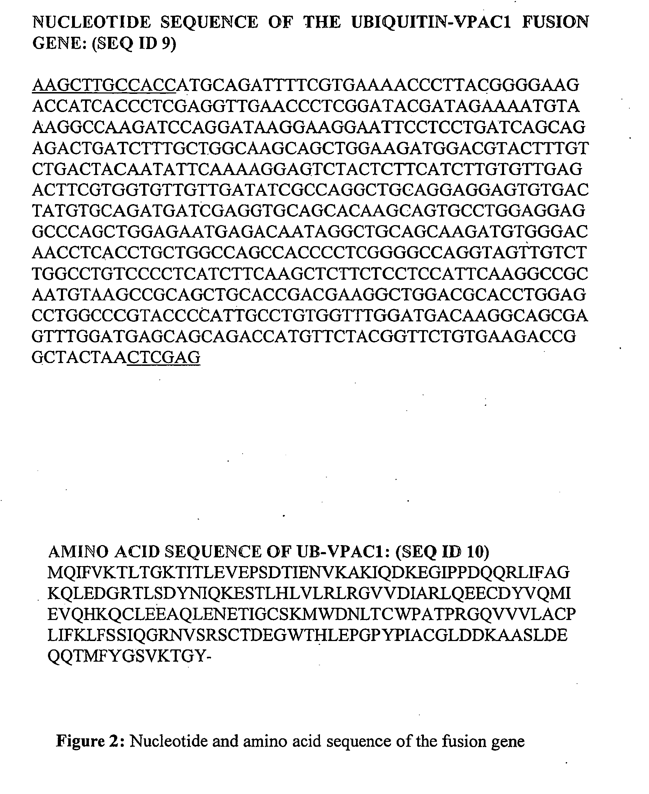

**Figure 2:**  shows the nucleotide and amino acid sequence of the fusion gene (SEQ ID 9 and SEQ ID 10 respectively).

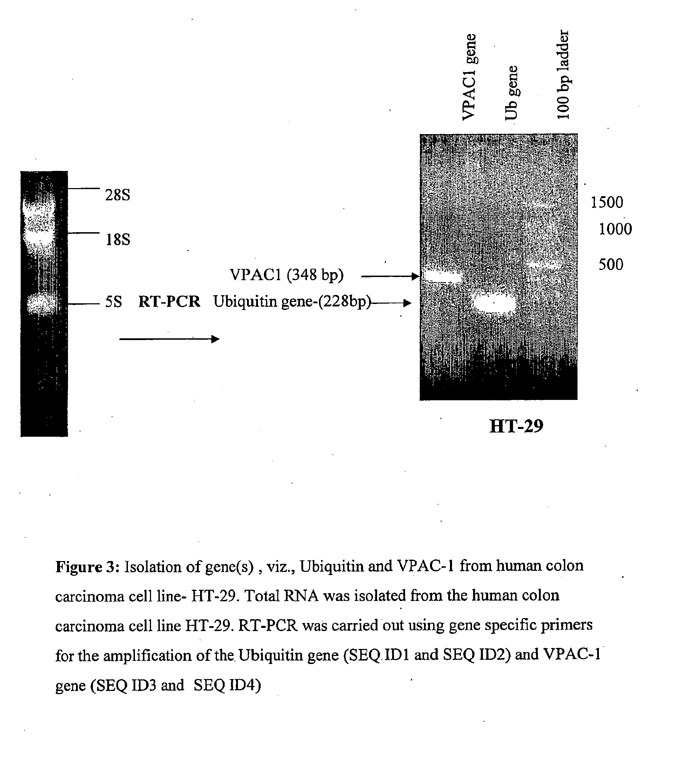

**Figure 3:**  shows the RT-PCR amplification of the Ubiquitin and VPAC1 gene(s) from the cDNA of HT-29 colon carcinoma cell line.

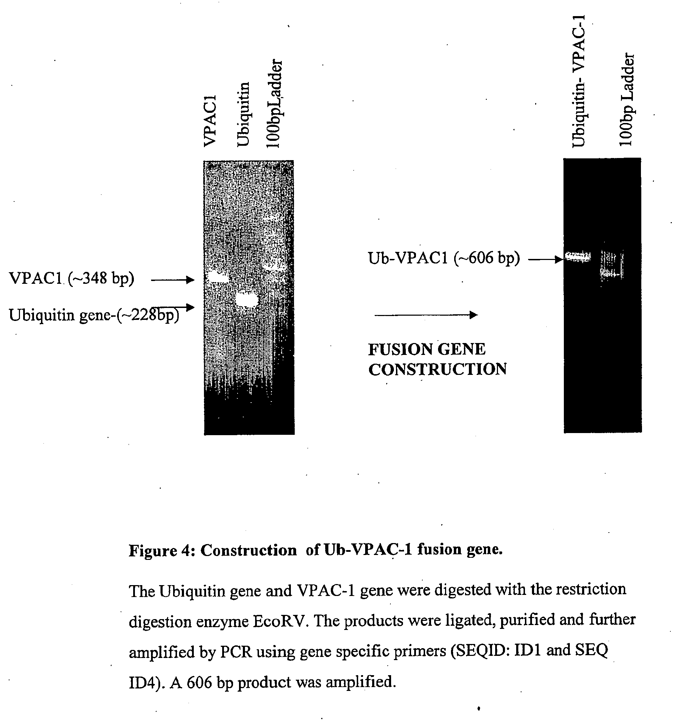

**Figure 4:**  shows the construction of the Ub-VPAC1 fusion gene by ligation of the individual fragments.

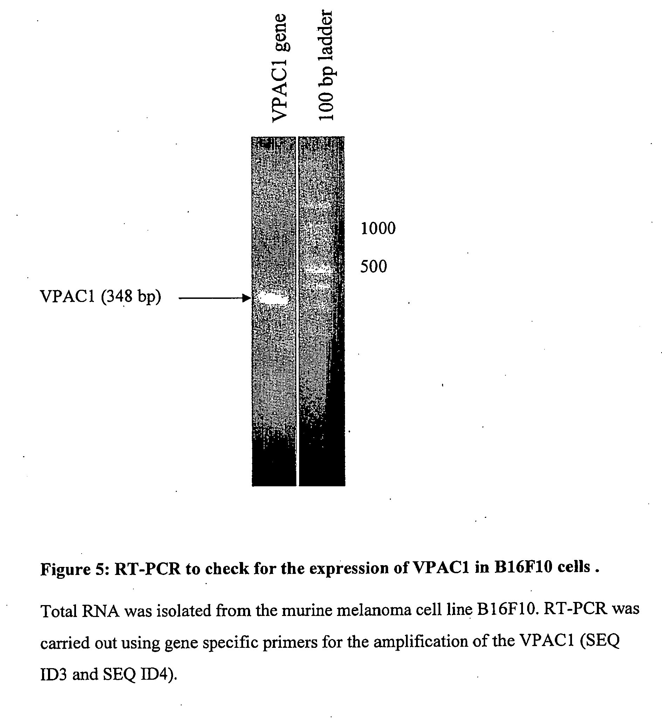

**Figure 5:**  shows the PCR amplification of the VPAC1 gene (from the cDNA of B 16F10 murine melanoma cell line.

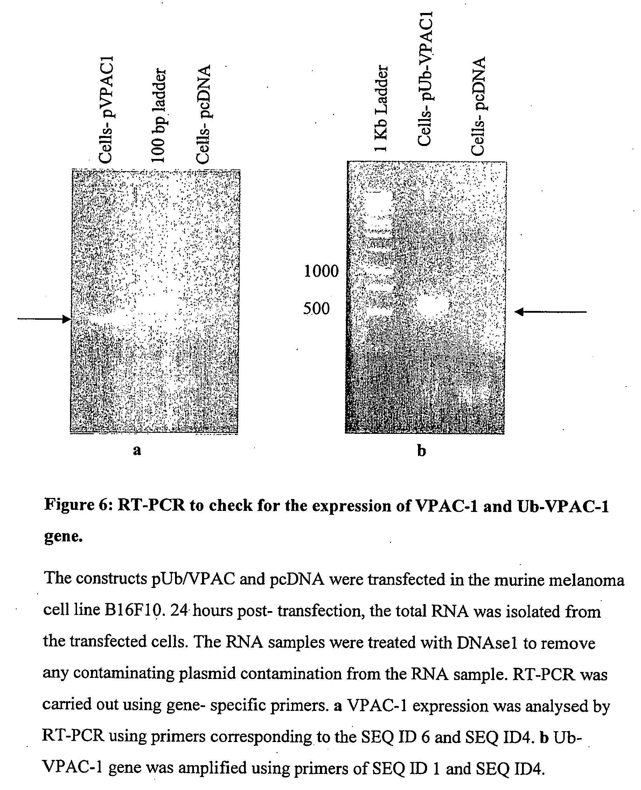

**Figure 6:**  shows the transfection and expression of VPAC1 and UbVPACI gene(s) in B16F10 murine melanoma cell line.

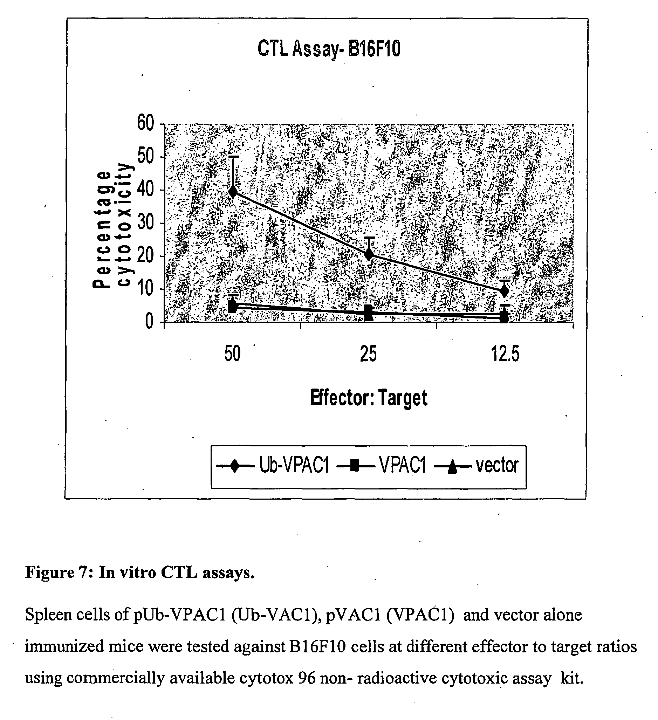

**Figure 7:**  shows the *in vitro* CTL assays for spleen cells isolated from Different experimental groups.

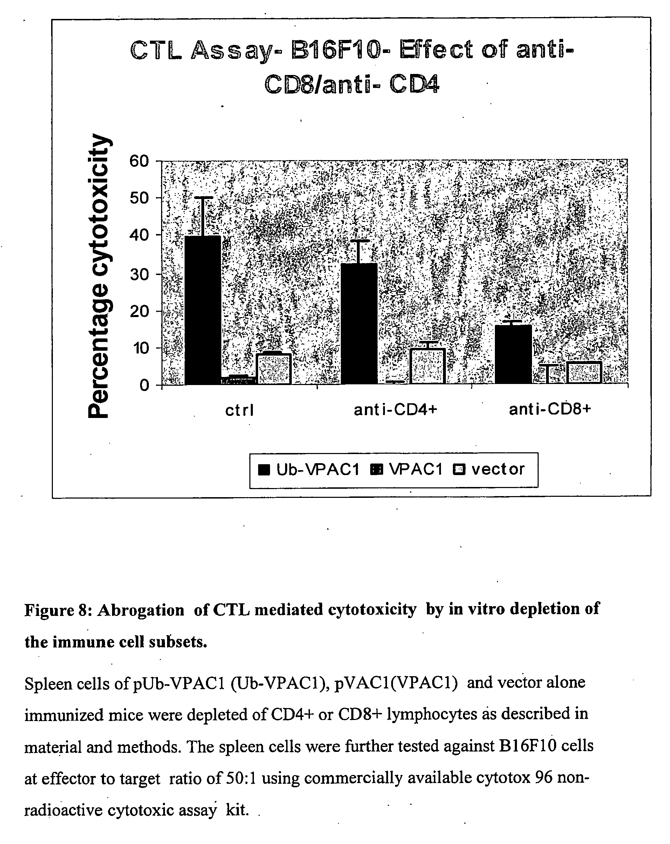

**Figure 8:**  shows the effect of *in vitro* depletion of the immune subsets on CTL mediated cytotoxicity.

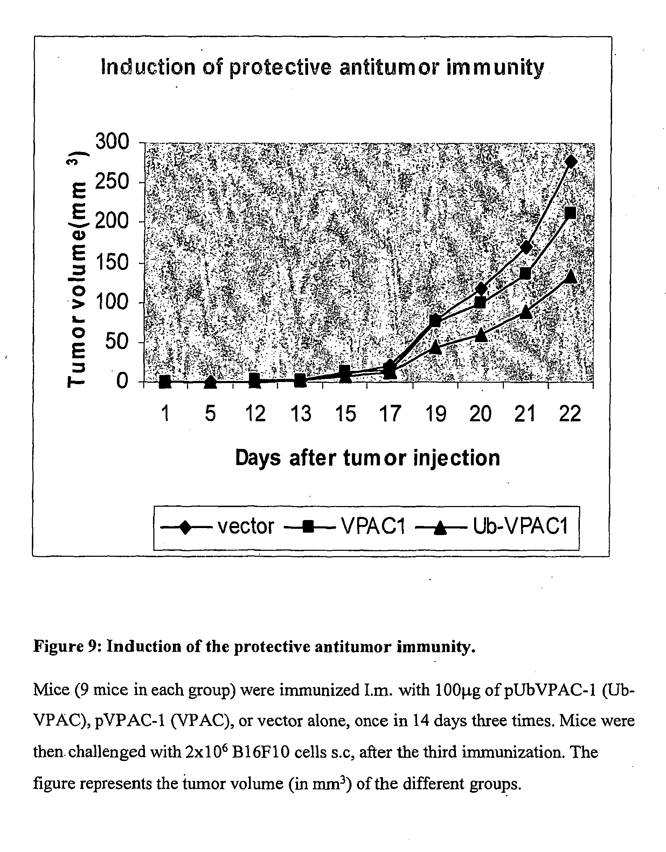

**Figure 9:**  shows the effect of DNA vaccination in induction of protective anti tumor immunity.

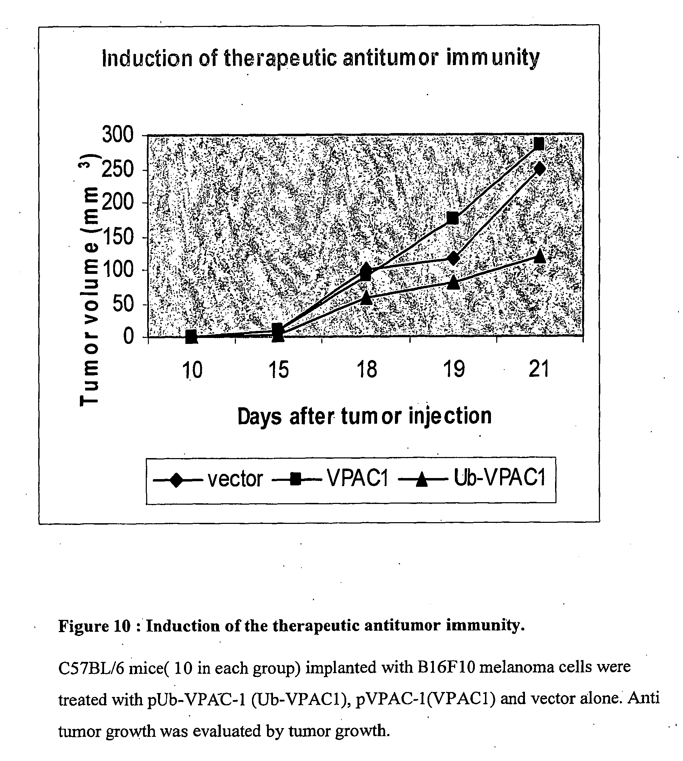

**Figure 10:**  shows the effect of DNA vaccination in induction of therapeutic anti tumor immunity

## DETAILED DESCRIPTION OF THE INVENTION

Growth factors (GF) and growth factor receptors (GFR) play an important role in the normal process of growth and differentiation [Annu. Rev. Biochem.,1993, 62, 453- 481]. Aberrations in the GFR signaling pathways can lead to abnormal growth and development. Functional mutations in growth factor receptors can lead to inherited diseases such as insulin- dependent diabetes (insulin receptor) [EMBO J., 1989, 8, 2509- 2517] and dwarfism /Achondroplasia (Fibroblast growth factor receptor 3) [Cell, 1994, 78, 335- 342]. Overexpression of transforming growth factor alpha (TGF$\alpha$) leads to non- neoplastic disorders like psoriasis [Science, 1989, 243, 811-814]. Some of the GFRs have been shown to play a critical role in the induction of cancer. The mechanism by which these receptors could contribute to tumorigenesis includes over expression of the receptors or their ligands and mutations on the receptors resulting in abnormal activity even in the absence of the ligand [Current Science, 2001, 81(5), 535-541]. Most of these GFR gene encode a Src family receptor tyrosine kinase with oncogenic potential and often triggers a cascade of cellular biochemical events leading to cell growth and differentiation in the presence of the binding- ligands [Current Science, 2001, 81(5), 535-541].

The overexpression of EGFR has been detected in many tumors, including non-small cell lung cancer (NSCLC), cancers of breast, bladder, and brain [J. Immunol., 2003, 170, 3162- 3170]. Insulin-like growth factors (IGF's) and their receptors have been found to be expressed in a wide range of tumors, including Wilm's tumor, liver cancer, lung cancer, breast cancer, etc [Current Science, 2001, 81(5), 535-541]. Similarly, FGF family of growth factors and their receptors have been shown to be overexpressd in pancreatic [Pancreas, 1998, 17, 169- 175] cancers and prostrate cancers [Clin. Cancer Res., 1999, 5, 1063-1071].

Several strategies aimed at blocking the mitogenic signaling pathway that is activated following ligand- receptor interactions have been evaluated. These include growth factor antagonist like suramin, monoclonal antibodies [Nature Biotech., 2005, 23, 1147- 1157] receptor dimerization inhibitors, protein tyrosine kinase inhibitors . (genistein, erbstatin, tyrphostins), antisense oligonucleotides and transcriptional inhibitors [Current Science, 2001, 81(5), 535-541]. Monoclonal antibodies raised against the receptors can also be used for targeting tumors overexpressing the tumors [J Immunol., 2003, 170, 3162- 3170] Erbitux and Herceptin are two major examples of this class of therapeutics targeting EGFR and Her2/neu respectively.

Another group of receptors, which have been shown to be highly overexpressed in cancer tissues are the vasoactive intestinal peptide receptors (VIP) receptors. A majority of human tumors, in particular the frequently occurring carcinomas, express VIP receptors [Cancer Res, 2000, 60, 3105- 3112]. Based on this high occurrence of tumoral VIP receptors, a number of potential clinical applications have been evaluated. First, it could be demonstrated that selected tumors, in particular, the VIP receptor-positive colorectal cancers, can be visualized in the patient by means of *in vivo* VIP receptor scintigraphy [N. Engl.J. Med., 1994, 331, 1116- 1121]. Moreover, several studies have reported an effect of VIP and PACAP analogues on tumor growth in animal tumor models, mediated by specific receptors [PNAS, 1993, 90, 4345- 4349]. Therefore, receptors for VIP may be of great importance for oncology in the field of oncotherapy.

Growth factor receptors are over expressed in cancer and serves as a good candidate for cancer vaccines. VPAC is a receptor to VIP. There are three different types of VPAC receptors, that include VPAC1, VPAC2, and PAC1 receptor that binds to pituitary adenylate cyclase activating peptide (PACAP) [J. Mol Med, 2002, 80,16-24]. Expression of VPAC1 receptors have been reported in most frequently occurring malignant neoplasms, such as cancers of the lung, stomach, colon, rectum, breast, prostate, pancreatic ducts, liver and urinary bladder [Cancer Res., 2000, 60, 3105-311]. In contrast to the ubiquitous expression of VPAC1 receptors in most human tumors, VPAC2 receptor expression has been seen in only few tumors. This includes the benign smooth muscle tumors and the leiomyomas [Endocrine Rev., 2003, 24(4), 389- 427]. PAC1 receptors are expressed predominantly in tumors originating from the neuronal and endocrine systems; this includes glial tumors, neuroblastomas, as well as various pituitary adenomas [Endocrine Rev., 2003, 24(4), 389-427].

VPAC1 is differentially expressed in high proportions in many cancer cells. Tumors expressing VPAC1 receptors include the most frequently occurring malignant epithelial neoplasms such as cancers of the lung, stomach, colon, rectum, breast, prostate, liver, melanoma and urinary bladder. So, VPAC-1 would have a role in targeting cancers of multiple organs.

Besides VIP, receptors of Somatostatin viz. sst1 sst2, sst3, sst4, and sst5, Bombesin and GRP receptors viz. neuromedin B receptor subtype (BB1), the GRP receptor subtype (BB2), the BB3 and BB4 subtypes, Substance P receptors viz. NK1, NK2, NK3, FGFR, and EFGR.are also found to be expressed in high proportions in many cancer cells.

Ubiquitin is a 76- amino acid polypeptide expressed in all eukaryotic cells and highly conserved from yeast to humans [FASEB J., 1997, 11, 1245- 1256]. The covalent attachment of Ub to cellular proteins mediates the degradation of both short- and long- lived proteins by the proteasome and also participates in the removal of abnormal and denatured proteins [Annu. Rev. Biochem., 1998, 67, 425- 479]. The Ub-proteasome pathway has an important role in a multitude of biological processes, including cell cycle progression, cell differentiation, signal transduction, DNA repair, apoptosis, and stress response [Bioessays, 2000, 22, 442- 451]. In addition, the system is involved in the production of peptides for antigen presentation by major histocompatibility complex (MHC) class I molecules.

In the first step of the pathway, Ub becomes linked to substrate proteins through the sequential addition of Ub enzymes forming an isopeptide bond between the C terminus of Ub and the ε-amino group of an internal lysine residue of the target protein. Ubiquitinylation begins with the ATP- dependent activation of Ub by an activating enzyme (E1), followed by transfer of Ub to an Ub- conjugating enzyme (E2). E2 delivers Ub to the substrate- specific Ub ligase E3, which transfers Ub to the protein to be degraded [Nat. Rev. Mol. Cel. Biol, 2001, 2, 169- 178]. After transfer of the first Ub moiety to the substrate protein, repetitive transfer of additional Ub molecules principally to Lys48 of the substrate-linked Ub yields a polyubiquitin chain [Science, 1989, 243, 1576-1583].

The second part of the pathway involves delivery of the polyubiquitinated substrate protein to the 26S proteasome, where the protein is degraded to smaller peptides and the polyubiquitin chain is converted back into monoubiquitin by Ub-specific hydrolases [Biochem. Biophyss. Res. Commun., 1999, 266, 633- 640]. After resulting peptides are transported into the endoplasmic reticulum, they associate there with MHC class I heavy and light chains. When properly assembled and loaded with peptides, the class I molecules move via the secretory pathway to the cell surface, where they are recognized by CD8+ T cells [Nature, 1993, 363, 552- 554].

In recent years, there has been interest in targeting key proteins to the proteasome via fusion to Ub to either down regulate protein function in cells or amplify T- cell responses against infectious agents and cancer cells [J.Cell Physiol., 2000,182, 1-11].

There are two primary Ub fusion approaches for proteasome targeting: the N-end rule and the UFD pathway. The first strategy involves the synthesis of an antigen that is transiently linked to Ub. The coding sequence of a vaccine antigen with a nonmethionine N-terminus is fused in frame to the C-terminus of the coding sequence of an Ub monomer. This fusion protein mimics a poly-Ub preprotein and is recognized by Ub hydrolases that normally cleave at the Ub C-terminus to create functional Ub monomers. Following cleavage of this conjugate, a non-conjugated vaccine antigen with an altered N-terminus is formed. The N-end rule dictates that a protein in this state is unstable and may not require ubiquitination to be degraded. It has been confirmed that in some systems this destabilized product is degraded rapidly and causes the induction of a protective antigen-specific CTL response in a lethal tumor challenge model. [J.Leuk Biol., 2000, 68, 793- 806]

A second fusion protein-encoding plasmid strategy involves the rapid degradation of noncleavable Ub conjugates. Although the previous system relied on protein destabilization following cleavage by Ub hydrolayses, this one takes advantage of the destabilizing properties of a fixed, uncleavable, Ub-conjugated protein. A particular base-pair mutation in Ub renders it resistant to cleavage, and it retains its normal function of tagging proteins for proteasome cleavage. Rapid degradation of this uncleavable conjugate has been demonstrated to enhance antigen-specific CTL responses, conferring viral protection at the expense of antibody production. Because of its rapid intracellular turnover, little protein is found in the cytoplasm at any one time, and none is secreted, which effectively abolishes its entry into the APC exogenous pathway.

Andersson et. al had carried out a detailed study on maximizing antigen targeting to the proteasome for gene-based vaccines [Mol. Therapy, 2004, 10(3), 432-466]. They had fused a series of engineered Ub genes to green fluorescent protein (GFP) and tested their ability to target GFP to the proteasome for enhanced antigen processing and CD8+ T- cell responses. Their studies showed that for the optimal degradation of an Ub fusion protein, the construct UbVV was the most promising. In this construct, the C- terminal glycine of the Ub was mutated to valine (G76V), followed by an incorporation of a valine at the N- terminal of the desired protein construct. The construct was found to be effective in mammalian cells and inhibit the cleavage of Ub from the fusion protein, thus mediating UFD targeting and inducing more potent T- cell mediated immune response compared to the non-modified constructs or constructs with substitutions other than "VV".

Zhang et. al. had shown for the first time that naked DNA vaccines encoding a ubiquitin- fused self- antigen preferentially induces the main effector CD8+ cells through efficient proteolysis mediated by the ubiquitin- proteasome pathway [Gene therapy, 2005, 12, 1049- 1057]. They showed that immunization with DNA vaccine containing ubiquitin gene fused with minigenes encoding cytotoxic T lymphocytes (CTL) epitopes of melanoma differentiation antigens broke self- tolerance and induced protective immunity to melanoma in C57BL/6J mice, as evaluated by tumor growth, survival rate and lung metastasis. Thus, fusion of ubiquitin to the self-antigen overexpressed in tumors seems to be an interesting strategy to overcome immunological tolerance.

Apart from the self- antigens, there are a number of other potential cancer targets that researchers are using for novel immunotherapeutic treatments. This includes the cell- cycle related protein p53, virally encoded proteins like human papilioma virus16 [HPV16]- encoded early antigen 6 and 7 (E6 and E7 respectively), or the Epstein- barr virus (EBV)- encoded latent membrane protein1 (LMP1), etc. But the major problem associated with these viral or mutated cellular oncogenes is their poor immunogenicity. For targeting this problem, Ub fusion of the oncogenes or the viral genes could be exploited.

Tellam et al had constructed a Ub- fusion in frame with the viral oncogene LMP1. This strategy not only increased the intracellular degradation rate of LMP1, but also resulted in complete inactivation of LMP-1 mediated cellular signaling and oncogenic potential, while at the same time enhanced the CD8+ T cell response to the antigen [Blood, 2003, 102 (13), 4535-4540]

With these observations we have designed a fusion gene comprising of suitably mutated sequences of ubiquitin (Ub) gene ligated to suitably modified gene sequence of the VPAC1 growth factor receptor or fragments thereof, over expressed in cancer cells. The ubiquitin tag will target the antigen gene towards proteasome leading to activation of MHC class I pathway and clonal growth of CD8+ killer T-cells that will provide both prophylactic and therapeutic immunity against cancer. Fusion gene inhibits the growth of cancer cells as well as prevents the growth of cancer cells and hence are i useful both as therapeutic and prophylactic agents for cancer therapy.

More specifically, the present invention is useful as a prophylactic agent especially in the form of a DNA vaccine for cancer therapy.

Therapeutic vaccines attempt to treat chronic infectious conditions by stimulating the immune system to more adequately defend the body in the situations when the patient is unable to develop an effective immune response. Therapeutic cancer vaccines aim to stop or retard the growth of existing tumors and also aim to prevent recurrence of cancer.

A prophylactic vaccine is a vaccine designed to prevent the growth of cancer. The vaccine may be administered prior to exposure to the pathogen, or after exposure to a pathogen but prior to the occurrence of disease. Prophylactic cancer vaccine could be given to an individual before cancer develops and could aim at stimulating the immune cells so that the tumor growth can be stopped or retarded on subsequent challenge.

In the present study, the modified gene sequence, coding for the extra cellular domain of VPAC1 is fused to the 3' terminal of the mutated Ub gene to effectively target the antigen (VPAC1) to the MHC- class I pathway. Expression vectors of pVPAC1 encoding full length extra cellular domain of VPAC1 and pUb-VPAC1 encoding modified ubiquitin (G76)-in frame with VPAC1 so as not to be cleaved by ubiquitin C- terminal hydrolases in cells were constructed. Figure-1 summarizes the strategy for the isolation and purification of the Ubiquitin and growth factor receptor gene and the construction of fusion gene is demonstrated.

In the synthetic fusion gene, C-terminus codon for glycine amino acid residue of the ubiquitin (gly76) has been substituted with codons for non-polar amino acids like valine to prevent cytosolic degradation of the fusion protein and to target the protein to the ubiquitin fusion degradation (UFD) pathway. But interestingly, in the mammalian system of proteasome targeting, the Ub fusion mediates most of its effect through UFD pathway rather than N- end rule [Mol. Therapy, 2004, 10(3), 432-466]. The mutated sequences of ubiquitin gene (Ub) is fused to the suitably modified gene sequence of the extracellular domain of the growth factor receptor viz. VPAC1, wherein a valine residue has been incorporated towards the N- terminus of the VPAC1 gene to enhance the targeting of the fusion protein to the UFD pathway and additional amino acids has been incorporated (in this case Asp, Ile) to generate a restriction enzyme site for proper linkage of the Ub and VPAC1 gene.

In our study, we have seen that VPAC-1 alone does not have any protective/therapeutic role against tumor cells, where as ubiquitinylated VPAC-1 shows significant immune response, as seen by both in vitro and in vivo studies.

For clarity of discussion, the specific composition, procedures and methods described herein are exemplified using VPAC1 as the target antigen. Analogous procedures and techniques are equally applicable for other antigens to be used as targets for the ubiquitin based therapy.

### Design of the Fusion Gene

The fusion gene of the present invention may be produced by fusion of mutated ubiquitin gene and the modified codon sequence of the extra cellular domain of the VPAC1 growth factor receptor, over expressed in cancer. In the present study, gene encoding the extra cellular domain of the VPAC1 growth factor receptor has been used as the target antigen. More specifically, gene encoding the extra cellular domain of VPAC1 has been used for the studies. Tumors expressing VPAC1 receptors include the most frequently occurring malignant epithelial neoplasms such as cancers of the lung, stomach, colon, rectum, breast, prostate, liver, melanoma and urinary bladder.

The fusion of the two- full-length coding sequences can be achieved by methods well known in the art. It is preferred that the fusion gene contains only the AUG translation initiation codon at the 5' end of the first coding sequence without the initiation codon of the second coding sequence to avoid the production of two encoded products.

For efficient translation of the gene, enhancer sequences composed of GCCACC has been placed at the 5' end of the N- terminal ubiquitin gene, upstream of the AUG initiation codon. C-terminus codon for glycine amino acid residue of the ubiquitin (gly76) has been substituted with codons for non-polar amino acids like valine to prevent cytosolic degradation of the fusion protein and to target the protein to the ubiquitin fusion degradation (UFD) pathway (SEQ ID No. 7). The mutated sequences of ubiquitin gene (Ub) is fused to the suitably modified gene sequence of the extracellular domain of the growth factor receptor viz. VPAC1, wherein a valine residue has been incorporated towards the N- terminus of the VPAC1 gene to enhance the targeting of the fusion protein to the UFD pathway and additional amino acids have been incorporated (in this case Asp, Ile) to generate a restriction enzyme site for proper linkage of the Ub and VPAC1 gene. SEQ ID No. 9 describes the complete nucleotide sequence of the fusion gene. SEQ ID No. 10 shows the deduced amino acid sequence of the fusion gene.

### Cloning and expression of the fusion gene

The fusion gene comprising of Ubiquitin and the coding sequence for the extra cellular domain of a VPAC1 gene may be cloned into an appropriate eukaryotic expression vector by using standard recombinant DNA techniques. The gene product itself may contain deletions, additions or substitutions of amino acid residues within a fusion sequence, which may result in silent changes, thus, producing a functionally equivalent protein. The recombinant vector used for the cloning of Ub-VPAC1 gene includes commercially available eukaryotic plasmids like pcDNA3.1HisA pcDNA3.1HisA is a 5.5 kb vector derived from pcDNA3 and designed for high-level stable and transient expression in mammalian hosts. High-level stable and non-replicative transient expression can be carried out in most mammalian cells. The pcDNA3 vectors contains human cytomegalovirus immediate-early (CMV) promoter for high-level expression in a wide range of mammalian cells, multiple cloning sites in the forward (+) and reverse (-) orientations to facilitate cloning, neomycin resistance gene for selection of stable cell lines, episomal replication in cells lines that are latently infected with SV40 or that express the SV40 large T antigen (e.g. COS-1, COS-7).

This is one example of a vector that can be used for cloning and expression of the fusion gene and derivatives thereof. Different vectors with various promoters and polyA signals, recombinant viruses, expression vectors for other species such as for example yeast, fungi, insect cells and plants can be used.

The plasmid constructs may be transfected *in vitro* in different types of mammalian cells and expression analysis can be done by various techniques, like, RT-PCR; immunofluorescence, immunoblots, ELISA, etc.

### In vitro and in vivo uses

The vaccine of the invention may be used against various cancer types overexpressing the VPAC1 receptor including cancers of lung, stomach, colon, rectum, breast, prostate, pancreas, liver and urinary bladder, etc. The vaccine comprising of ubiquitin gene fused with the extracellular domain of the VPAC1 gene will be able to break the self-tolerance against the self-antigen through effective antigen processing and presentation. The ubiquitin tag will target the antigen gene towards proteasome leading to activation of MHC class I pathway and clonal growth of CD8+ killer T-cells that will provide both prophylactic and therapeutic immunity against cancer.

The vaccine provided by this invention may be administered subcutaneously, intramuscularly, intradermally, or into an organ. The vaccine also may be injected directly into the tumor to enhance or induce immunity. Intramuscular injection has been shown in the past to be an important delivery route for induction of immunity. Skeletal muscle has

EP 1 962 891 B1

properties such as high vascularization and multi-nucleation. In addition, it is nonreplicating and capable of expressing recombinant proteins. These properties are advantageous for gene therapy. One theory of the mechanism of how muscle presents the protein and induces immune response is that recombinant protein is produced and released into the vascular network of the muscle and eventually presented by professional antigen-presenting cells such as dendritic cells, myoblasts, or macrophages infiltrating the muscle. Another suggestion is that at the injection site muscle injury induces myoblast proliferation and activation of infiltrating macrophages or dendritic-like cells, and they then present antigens through MHC class II antigen. Thus, other tissues, which have similar qualities, also would be good delivery sites for the vaccine. The DNA vaccine of the present invention can also be administered by gene gun technology. The route of delivery of the vaccine has been exemplified using intramuscular route.

DNA vaccine of the present invention may be administered serially or in combination with other therapeutics used in the treatment of cancer and other related diseases. These therapeutics include IFN-alpha, IFN-beta, interleukin-1, interleukin-2, tumor necrosis factor, macrophage colony stimulating factor, macrophage activation factor, lymphotoxin, fibroblast growth factor, etc (derived from natural sources or expressed recombinantly). Alternatively, the DNA vaccine may be administered serially or in combination with conventional chemotherapeutic agents such as 5-fluoro uracil; paclitaxel; etoposide; carboplatin; cisplatin; topotecan, methatroxate, etc. and/or radiotherapy. Such combination therapies may advantageously utilize less than conventional dosages of those agents, or involve less radical regimens, thus avoiding any potential toxicity or risks associated with those therapies.

Genetic immunization with ubiquitin fused target gene will be primarily used a therapeutic vaccine. It may be used in patients suffering from various forms of cancers such as T- and B- cell lymphoproliferative diseases, ovarian cancer, breast cancer, pancreatic cancer, head and neck cancer, squamous cell carcinomas, gastrointestinal cancer, and non-small cell lung cancer, etc, which have been reported to show over expression of VPAC 1 receptor.

The vaccine will also have a prophylactic role in patients of high-risk group. This group comprises of the families of cancer patients, where the hereditary linkage has been established, and/ or patients where the recurrence of cancer is suspected.

For a better understanding of the invention, the following examples are set forth. Throughout these examples, all molecular cloning reactions were carried out according to methods in Molecular Cloning--A laboratory Manual, 3rd, Cold Spring Harbor Press, 2001.


**Example 1**


**Construction Of Fusion Gene**

A cDNA encoding for human Ub and VPAC1 gene was obtained by reverse transcriptase polymerase chain reaction (RT-PCR), using RNA isolated from HT-29 cells specific primers for Ub (SEQ ID 1 and SEQ ID 2) and VPAC1 (SEQ ID 3 and SEQ ID 4) were used to amplify the genes selectively from cDNA of HT29 colon cancer cells. The reaction condition of the PCR was; 97° C for 3 min, followed by thirty cycles of 95°C at 1 min, 60° C for 2 min, 72° for 30 sec. After this a final step of elongation at 72° C for 7 min was followed by cooling at 4° C.

Plasmid containing human VPAC1 was constructed as: VPAC1 cDNA was amplified by PCR using sense (SEQ ID 5) and antisense (SEQ ID 4) primers. PCR product of VPAC1 cDNA was inserted into HindIII and Xho1 site of pcDNA3.1HisA vector (Invitrogen, San Diego, CA, USA). The resulting plasmid, designated pVPAC1, contained the human VPAC1 coding sequence.

A plasmid for the expression of Ub fused VPAC1 (extra cellular domain) gene under the control of the CMV promoter was constructed as shown in (Figure-1). The amplified Ub and VPAC1 gene(s) were digested with EcoRV and ligated. The fusion gene fragment was amplified by PCR using the flanking primers and with HindIII and Xho1. The digested fusion fragment was ligated to pcDNA3.1HisA expression vector. The resulting plasmid, designated pUb-VPAC1, contained the human VPAC1 coding sequence fused to the 3' end of the human Ub gene. The C-terminus codon for glycine amino acid residue of the ubiquitin (gly76) has been substituted with codons for non-polar amino acids like valine to prevent cytosolic degradation of the fusion protein and to target the protein to the ubiquitin fusion degradation (UFD) pathway (Figure-2). The mutated sequences of ubiquitin gene (Ub) is fused to the suitably modified gene sequence of the extracellular domain of the growth factor receptor viz. VPAC1, wherein a valine residue has been incorporated towards the N- terminus of the VPAC1 gene to enhance the targeting of the fusion protein to the UFD pathway and additional amino acids has been incorporated (in this case Asp, Ile) to generate a restriction enzyme site for proper linkage of the Ub and VPAC1 gene. Giga prep was carried out to get sufficient plasmid DNA of pcDNA3.1HisA, pVPAC1 and pUb-VPAC1 for further studies..

### Example 2

**Expression Of The Fusion Gene**

The expression of the genes (Ub-VPAC1 and VPAC1) was confirmed by RT-PCR. Transfection was carried out using commercially available Lipofectamine reagent (Invitrogen). Transfection was carried out in murine melanoma B16F10 cells. B16F10 cells were plated in six well plates at 90 % confluency in DMEM media without antibiotics. $4\mu g$ of plasmid DNA of each construct, viz. pcDNA; pVPAC1 and pUb-VPAC1 was diluted in 250uL of DMEM media without serum and antibiotics. 10 $\mu$l of lipofectamine™ 2000 reagent (Invitrogen) was also diluted in a similar way. After 5 minutes of incubation at room temperature, diluted DNA and lipofectamine were mixed together to form a complex. After 20 minutes of incubation at room temperature the complex was added to the cells in 2 ml of DMEM media without sera and antibiotics. Plate was incubated inside the $CO_2$ incubator for 6 hours and then media was replaced with complete DMEM media. 24 hours post- transfection, 1 ml of Trizol reagent (Invitrogen) was added to the cells after a PBS washing for extraction of RNA. The total RNA was extracted using chloroform-isopropyl alcohol method. Finally, the pellet was washed with 70% ethanol and dried on a speed- vac. Control RNA from untreated cells was also prepared in a similar fashion. To remove any contaminating plasmid, the RNA was treated with DNase 1.

RT PCR was carried out using one step RT PCR Kit (Qiagen) according to manufacturer's instructions. Briefly, $1\mu g$ of each type of RNA (pVPAC1 and pUb-VPAC1) was taken and mixed with specific primers of pVPAC1 (SEQ ID 6 and SEQ ID 4) and pUb-VPAC1 (SEQ ID 1 and SEQ ID 4) respectively Respective pcDNA control was also taken. The PCR amplified samples were analyzed on a 1.5 % agarose gel.

### Example 3

**CTL activity**

To investigate the tumor cytotoxicity of the constructs, female C57BL/6J black mice (6-8 week old) were immunized on hind limbs by intramuscular injections of plasmid constructs (pUb-VPAC1, pVPAC1, and vector alone). For each type of plasmid, 3 mice were used in each treatment group. The mice were immunized 3 times, at 14-days intervals, with 100 $\mu$g of plasmid DNA. 2-weeks after the final immunization, mice were euthanized. Spleen was isolated. The spleen cells were washed extensively with RPMI media. The cells were further treated with 0.1M ammonium chloride solutions for the lysis of RBCs. The cells were washed with the RPMI media, counted using a haemocytometer and used further as effector cells. The target cell line, B16F10 was plated on 96-well plate at a density of 7500 cells/well. After 2 hours, the effector cells were added and the co- cultures were incubated for another 4 hr. The effector to target ratio (E: T) was taken at 50:1, 25:1, 12.5: 1 and 6.25:1. Promega (Madison WI) Cyto Tox 96 non- radioactive cytotoxicity assay kit was used for measurement of cell lysis. Assay was carried out according to manufacturer's instructions. Percentage of lysis was calculated using the following formula:

$$\% \text{ lysis} = \frac{\text{experimental- target spontaneous}}{\text{target maximum- target spontaneous}} \times 100$$

In the cytotoxicity inhibition assays, effector cells were treated with murine mAb (anti-CD4 and anti-CD8), each used at a concentration of $10\mu g/ml$ for 30 min in ice. Following this incubation, the cells were washed three to four times and resuspended in minimal volume of RPMI media and used for the cytotoxicity assay. The effector to target ratio was maintained at 50:1.

### Example 4

### *In Vivo* Gene Transfer And Implantation Of Melanoma Cells

*a. Prophylactic effects of DNA vaccine:*

6- 8-week female C57BL/6J mice (9 in each group) were immunized intramuscularly three times, at 14- days interval, with 100-$\mu$g plasmid. A week after the final immunization, mice were challenged sub-cutaneously with $2x10^6$ B16F10 melanoma cells, prepared in 0.1ml PBS. Tumor size was measured daily using a vernier caliper and was calculated as using the formula $(d^2xD)/2$, where 'd' represents the smaller diameter of the tumor and 'D'-represents the larger diameter of the tumor.

*b. Therapeutic effects of DNA vaccine:*

For therapeutic trials, C57BL/6J female (6-8weeks old, 10 in each group) mice were implanted with B16F10 cells (2x10$^6$; subcutaneous) before vaccination (day 0). Treatment with pVPAC1, pUb-VPAC1 or vector plasmid commenced on day 1, and was repeated twice a week for a fortnight. Each group consisted of 10 mice. A total of 500µg plasmid was administered into each mouse for one treatment. Tumor size was measured as described above.

**SEQUENCE IDENTITIES:**

| Seq ID | Sequence | Sequence Occurrence (Artificial / Natural) | Source of the sequence |
|---|---|---|---|
| 1 | 5'AATAAGCTTGCCACCATGCAGAT TTTCGTGAAAACCCT 3' | Artificially generated oligonucleotide (sense primer) to amplify the human Ubiquitin gene (SEQ ID 7) from human carcinoma cell line. | NA |
| 2 | 5'AATGATATCAACAACACCACGAA GTCTCAACACAAGA3' | Artificially generated oligonucleotide (antisense primer) to amplify the human Ubiquitin gene (SEQ ID 7) from human carcinoma cell line. | NA |
| 3 | 5'AATGATATCGCCAGGCTGCAGGA GGAGT 3' | Artificially generated oligonucleotide (sense primer) to amplify the human VPAC-1 gene (SEQ ID 8) from human carcinoma cell line. | NA |
| 4 | 5'AATCTCGAGTTAGTAGCCGGTCT TCACAGAAC 3' | Artificially generated oligonucleotide (antisense primer) to amplify the human VPAC-1 gene (SEQ ID 8) from human carcinoma cell line. | NA |
| 5 | 5'AATAAGCTTGCCACCATGGCCAG GCTGCAGGAGGAGT 3' | Artificially generated oligonucleotide (sense primer) to amplify the human VPAC-1 gene (SEQ ID 8) from human carcinoma cell line. | NA |
| 6 | 5'CGCAAATGGGCGGTAGGCGTG 3' | Naturally occurring oligonucleotide (sense primer) corresponding to the 5' end of the CMV promoter. | Cytomegalovirus |

(continued)

| Seq ID | Sequence | Sequence Occurrence (Artificial / Natural) | Source of the sequence |
|---|---|---|---|
| 7 | 5'ATGCAGATTTTCGTGAAAACCCT TACGGGGAAGACCATCACCCTCGA GGTTGAACCCTCGGATACGATAGA AAATGTAAAGGCCAAGATCCAGGA TAAGGAAGGAATTCCTCCTGATCA GCAGAGACTGATCTTTGCTGGCAA GCAGCTGGAAGATGGACGTACTTT GTCTGACTACAATATTCAAAAGGA GTCTACTCTTCATCTTGTGTTGAGA CTTCGTGGTGGT3' | Naturally occurring nucleotide sequence corresponding to the ubiquitin gene amplified from a human carcinoma cell line using SEQ ID 1 and 2. | Human |
| 8 | 5'GCCAGGCTGCAGGAGGAGTGTGA CTATGTGCAGATGATCGAGGTGCA GCACAAGCAGTGCCTGGAGGAGGC CCAGCTGGAGAATGAGACAATAGG CTGCAGCAAGATGTGGGACAACCT CACCTGCTGGCCAGCCACCCCTCG GGGCCAGGTAGTTGTCTTGGCCTGT CCCCTCATCTTCAAGCTCTTCTCCT CCATTCAAGGCCGCAATGTAAGCC GCAGCTGCACCGACGAAGGCTGGA CGCACCTGGAGCCTGGCCCGTACC CCATTGCCTGTGGTTTGGATGACAA GGCAGCGAGTTTGGATGAGCAGCA GACCATGTTCTACGGTTCTGTGAAG ACCGGCTAC3' | Naturally occurring nucleotide sequence corresponding to the extracellular domain of VPAC-1 gene amplified from a human carcinoma cell line using SEQ ID 3 and 4. | Human |
| 9 | 5'<u>AAGCTTGCCACC</u>ATGCAGATTTT CGTGAAAACCCTTACGGGGAAGAC CATCACCCTCGAGGTTGAACCCTC GGATACGATAGAAAATGTAAAGGC CAAGATCCAGGATAAGGAAGGAAT TCCTCCTGATCAGCAGAGACTGAT CTTTGCTGGCAAGCAGCTGGAAGA TGGACGTACTTTGTCTGACTACAAT ATTCAAAAGGAGTCTACTCTTCATC TTGTGTTGAGACTTCGTGGTGTTGT TGATATCGCCAGGCTGCAGGAGGA | Artificially generated fusion gene, constructed by recombinant DNA technology, encoding the human ubiquitin protein fused with the extra cellular domain of human VPAC-1 protein. (With suitable modifications, as discussed in the text). Underlined region depicts the non-coding region of the construct. | Human |

(continued)

| Seq ID | Sequence | Sequence Occurrence (Artificial / Natural) | Source of the sequence |
|--------|----------|---------------------------------------------|------------------------|
| | GTGTGACTATGTGCAGATGATCGA GGTGCAGCACAAGCAGTGCCTGGA GGAGGCCCAGCTGGAGAATGAGAC AATAGGCTGCAGCAAGATGTGGGA CAACCTCACCTGCTGGCCAGCCAC CCCTCGGGGCCAGGTAGTTGTCTTG GCCTGTCCCCTCATCTTCAAGCTCT TCTCCTCCATTCAAGGCCGCAATGT AAGCCGCAGCTGCACCGACGAAGG CTGGACGCACCTGGAGCCTGGCCC GTACCCCATTGCCTGTGGTTTGGAT GACAAGGCAGCGAGTTTGGATGAG CAGCAGACCATGTTCTACGGTTCTG TGAAGACCGGCTACTAACTCGAG3' | | |
| 10 | MQIFVKTLTGKTITLEVEPSDTIENVK AKIQDKEGIPPDQQRLIFAGKQLEDG RTLSDYNIQKESTLHLVLRLRGVVDI ARLQEECDYVQMIEVQHKQCLEEAQ LENETIGCSKMWDNLTCWPATPRGQ VVVLACPLIFKLFSSIQGRNVSRSCTD EGWTHLEPGPYPIACGLDDKAASLD EQQTMFYGSVKTGY- | Artificially generated fusion protein, comprised of amino acid sequence corresponding to the human ubiquitin protein fused to the extra cellular domain of human VPAC-1 protein. (With suitable modifications, as discussed in the text) | Human |

**Claims**

1. A synthetic fusion gene comprising a sequence composed of an ubiquitin gene fused to a gene sequence encoding the extracellular domain of vasoactive intestinal receptor type 1, which is over expressed in cancer of various origins.

2. A synthetic fusion gene as claimed in claim 1 wherein the 3' end of the ubiquitin gene encoding for Gly76 is mutated to encode for a non-polar amino acid.

3. A synthetic fusion gene as claimed in claim 2 wherein the encoded non-polar amino acid is selected from Val, Ile, Ala or Leu.

**4.** A synthetic fusion gene as claimed in claim 3 wherein the encoded non-polar amino acid is Val.

**5.** A synthetic fusion gene as claimed in any one of claims 1 to 4 wherein the gene sequence of the extracellular domain of growth factor receptor is modified at its 5' end by addition of a codon encoding for non-polar amino acids.

**6.** A synthetic fusion gene as claimed in claim 5 wherein the codon encodes for a non-polar amino acid selected from Val, Ile, Ala or Leu.

**7.** A synthetic fusion gene as claimed in claim 6 wherein the encoded non-polar amino acid is Val.

**8.** A synthetic fusion gene as claimed in any preceding claim having a sequence of SEQ ID NO: 9.

**9.** A synthetic fusion gene as claimed in any preceding claim which is synthesized by recombinant DNA technology.

**10.** A DNA vaccine comprising a sequence composed of an ubiquitin gene fused to a gene sequence encoding the extracellular domain of vasoactive intestinal receptor type 1, which is over expressed in cancer of various origins.

**11.** A DNA vaccine as claimed in claim 10, wherein the sequence is as defined in any one of claims 2 to 9.

**12.** A synthetic fusion gene or DNA vaccine as claimed in any preceding claim for use in therapy.

**13.** A synthetic fusion gene or DNA vaccine as claimed in any one of claims 1 to 11 for use in the therapeutic or prophylactic treatment of cancer.

**14.** Use of the synthetic fusion gene or DNA vaccine as claimed in any one of claims 1 to 11 in the manufacture of a medicament for use in the therapeutic or prophylactic treatment of cancer.

**15.** A synthetic fusion gene or DNA vaccine of claim 13 or use of the synthetic fusion gene or DNA vaccine of claim 14 wherein said cancer is selected from colon, rectum, lung, breast, brain, pancreas, prostrate, liver, gastrointestinal, thyroid, ovary, head and neck or kidney cancer or from melanomas, neuroblastoma, glioblastoma, leukemia and lymphomas.

**Patentansprüche**

**1.** Synthetisches Fusionsgen, das eine Sequenz enthält, die aus einem Ubiquitin-Gen zusammengesetzt ist, das an eine Gensequenz fusioniert ist, die die extrazelluläre Domäne eines vasoaktiven intestinalen Rezeptors vom Typ 1, der bei Krebs unterschiedlichen Ursprungs übermäßig hervortritt bzw. exprimiert wird, codiert.

**2.** Synthetisches Fusionsgen nach Anspruch 1, wobei das 3'-Ende des Ubiquitin-Gens, das Gly76 codiert, mutiert ist, um eine nichtpolare Aminosäure zu codieren.

**3.** Synthetisches Fusionsgen nach Anspruch 2, wobei die codierte nichtpolare Aminosäure ausgewählt ist aus Val, Ile, Ala oder Leu.

**4.** Synthetisches Fusionsgen nach Anspruch 3, wobei die codierte nichtpolare Aminosäure Val ist.

**5.** Synthetisches Fusionsgen nach einem der Ansprüche 1 bis 4, wobei die Gensequenz der extrazellulären Domäne des Wachstumsfaktorrezeptors an ihrem 5'-Ende durch Addition eines Kodons, das nichtpolare Aminosäuren codiert, modifiziert ist.

**6.** Synthetisches Fusionsgen nach Anspruch 5, wobei das Kodon eine nichtpolare Aminosäure codiert, die ausgewählt ist aus Val, Ile, Ala oder Leu.

**7.** Synthetisches Fusionsgen nach Anspruch 6, wobei die codierte nichtpolare Aminosäure Val ist.

**8.** Synthetisches Fusionsgen nach einem vorhergehenden Anspruch, das eine Sequenz SEQ ID NO: 9 besitzt.

**9.** Synthetisches Fusionsgen nach einem vorhergehenden Anspruch, das durch eine DNA-Rekombinationstechnologie synthetisiert ist.

**10.** DNA-Impfstoff, der eine Sequenz enthält, die aus einem Ubiquitin-Gen zusammengesetzt ist, das an eine Gensequenz fusioniert ist, die die extrazelluläre Domäne eines vasoaktiven intestinalen Rezeptors vom Typ 1, der bei Krebs unterschiedlichen Ursprungs übermäßig hervortritt bzw. exprimiert wird, codiert ist.

**11.** DNA-Impfstoff nach Anspruch 10, wobei die Sequenz wie in einem der Ansprüche 2 bis 9 angegeben definiert ist.

**12.** Synthetisches Fusionsgen oder DNA-Impfstoff nach einem vorhergehenden Anspruch zur therapeutischen Verwendung.

**13.** Synthetisches Fusionsgen oder DNA-Impfstoff nach einem der Ansprüche 1 bis 11 zur Verwendung bei der therapeutischen oder prophylaktischen Behandlung von Krebs.

**14.** Verwendung des synthetischen Fusionsgens oder des DNA-Impfstoffs nach einem der Ansprüche 1 bis 11 bei der Herstellung eines Medikaments zur Verwendung bei der therapeutischen oder prophylaktischen Behandlung von Krebs.

**15.** Synthetisches Fusionsgen oder DNA-Impfstoff nach Anspruch 13 oder Verwendung des synthetischen Fusionsgens oder des DNA-Impfstoffs nach Anspruch 14, wobei der Krebs ausgewählt ist aus Dickdarmkrebs, Mastdarmkrebs, Lungenkrebs, Brustkrebs, Hirnkrebs, Bauchspeicheldrüsenkrebs, Prostatakrebs, Leberkrebs, Magen-Darm-Krebs, Schilddrüsenkrebs, Eierstockkrebs, Kopf- und Hals- oder Nierenkrebs oder aus Melanomen, Neuroblastomen, Glioblastomen, Leukämie und Lymphomen.

**Revendications**

**1.** Gène de fusion synthétique comportant une séquence composée d'un gène d'ubiquitine fusionné avec une séquence de gènes codant le domaine extracellulaire du récepteur intestinal vasoactif de type 1, qui est surexprimé dans un cancer d'origines diverses.

**2.** Gène de fusion synthétique tel que revendiqué dans la revendication 1, dans lequel l'extrémité 3' du gène d'ubiquitine codant pour Gly76 est muté afin de coder pour un acide aminé non polaire.

**3.** Gène de fusion synthétique tel que revendiqué dans la revendication 2, dans lequel l'acide aminé non polaire codé est choisi parmi Val, Ile, Ala ou Leu.

**4.** Gène de fusion synthétique tel que revendiqué dans la revendication 3, dans lequel l'acide aminé non polaire codé est Val.

**5.** Gène de fusion synthétique tel que revendiqué dans l'une quelconque des revendications 1 à 4, dans lequel la séquence de gènes du domaine extracellulaire du récepteur de facteur de croissance est modifiée au niveau de son extrémité 5' par ajout d'un codon codant pour des acides aminés non polaires.

**6.** Gène de fusion synthétique tel que revendiqué dans la revendication 5, dans lequel le codon code pour un acide aminé non polaire choisi parmi Val, Ile, Ala ou Leu.

**7.** Gène de fusion synthétique tel que revendiqué dans la revendication 6, dans lequel l'acide aminé non polaire codé est Val.

**8.** Gène de fusion synthétique tel que revendiqué dans l'une quelconque des revendications précédentes, ayant une séquence de SEQ ID N° : 9.

**9.** Gène de fusion synthétique tel que revendiqué dans l'une quelconque des revendications précédentes qui est synthétisé par une technologie d'ADN recombinant.

**10.** Vaccin à ADN comportant une séquence composée d'un gène d'ubiquitine fusionné avec une séquence de gènes codant le domaine extracellulaire du récepteur intestinal vasoactif de type 1, qui est surexprimé dans un cancer d'origines diverses.

11. Vaccin à ADN tel que revendiqué dans la revendication 10, dans lequel la séquence est telle que définie dans l'une quelconque des revendications 2 à 9.

12. Gène de fusion synthétique ou vaccin à ADN tels que revendiqués dans l'une quelconque des revendications précédentes pour une utilisation en thérapie.

13. Gène de fusion synthétique ou vaccin à ADN tels que revendiqués dans l'une quelconque des revendications 1 à 11 pour une utilisation dans le traitement thérapeutique ou prophylactique d'un cancer.

14. Utilisation du gène de fusion synthétique ou du vaccin à ADN tels que revendiqués dans l'une quelconque des revendications 1 à 11 dans la fabrication d'un médicament destiné à une utilisation dans le traitement thérapeutique ou prophylactique d'un cancer.

15. Gène de fusion synthétique ou vaccin à ADN selon la revendication 13 ou utilisation du gène de fusion synthétique ou du vaccin à ADN selon la revendication 14, dans lequel ledit cancer est choisi parmi le cancer du colon, du rectum, du poumon, du sein, du cerveau, du pancréas, de la prostate, du foie, gastro-intestinal, de la thyroïde, des ovaires, de la tête et du cou ou du rein, ou parmi des mélanomes, des neuroblastomes, des glioblastomes, une leucémie et des lymphomes.

**Mammalian Cell Line**

RNA Isolation

**First strand cDNA synthesis**

PCR using **gene specific** primers

**UBIQUITIN**                    VPAC1

HindIII ▉▉▉ EcoRV          EcoRV ▉▉▉ Xho1

EcoRV digestion                    EcoRV digestion·

FUSION GENE

UBIQUITIN    VPAC1

HindIII ▉▉▉ Xho1

EcoRV

**Figure 1:** Strategy for the construction of FUSION GENE

## NUCLEOTIDE SEQUENCE OF THE UBIQUITIN-VPAC1 FUSION GENE: (SEQ ID 9)

AAGCTTGCCACCATGCAGATTTTCGTGAAAACCCTTACGGGGAAG
ACCATCACCCTCGAGGTTGAACCCTCGGATACGATAGAAAATGTA
AAGGCCAAGATCCAGGATAAGGAAGGAATTCCTCCTGATCAGCAG
AGACTGATCTTTGCTGGCAAGCAGCTGGAAGATGGACGTACTTTGT
CTGACTACAATATTCAAAAGGAGTCTACTCTTCATCTTGTGTTGAG
ACTTCGTGGTGTTGTTGATATCGCCAGGCTGCAGGAGGAGTGTGAC
TATGTGCAGATGATCGAGGTGCAGCACAAGCAGTGCCTGGAGGAG
GCCCAGCTGGAGAATGAGACAATAGGCTGCAGCAAGATGTGGGAC
AACCTCACCTGCTGGCCAGCCACCCCTCGGGGCCAGGTAGTTGTCT
TGGCCTGTCCCCTCATCTTCAAGCTCTTCTCCTCCATTCAAGGCCGC
AATGTAAGCCGCAGCTGCACCGACGAAGGCTGGACGCACCTGGAG
CCTGGCCCGTACCCCATTGCCTGTGGTTTGGATGACAAGGCAGCGA
GTTTGGATGAGCAGCAGACCATGTTCTACGGTTCTGTGAAGACCG
GCTACTAACTCGAG

## AMINO ACID SEQUENCE OF UB-VPAC1: (SEQ ID 10)

MQIFVKTLTGKTITLEVEPSDTIENVKAKIQDKEGIPPDQQRLIFAG
KQLEDGRTLSDYNIQKESTLHLVLRLRGVVDIARLQEECDYVQMI
EVQHKQCLEEAQLENETIGCSKMWDNLTCWPATPRGQVVVLACP
LIFKLFSSIQGRNVSRSCTDEGWTHLEPGPYPIACGLDDKAASLDE
QQTMFYGSVKTGY-

**Figure 2:** Nucleotide and amino acid sequence of the fusion gene

**Figure 3:** Isolation of gene(s) , viz., Ubiquitin and VPAC-1 from human colon carcinoma cell line- HT-29. Total RNA was isolated from the human colon carcinoma cell line HT-29. RT-PCR was carried out using gene specific primers for the amplification of the Ubiquitin gene (SEQ ID1 and SEQ ID2) and VPAC-1 gene (SEQ ID3 and SEQ ID4)

**Figure 4: Construction of Ub-VPAC-1 fusion gene.**

The Ubiquitin gene and VPAC-1 gene were digested with the restriction digestion enzyme EcoRV. The products were ligated, purified and further amplified by PCR using gene specific primers (SEQID: ID1 and SEQ ID4). A 606 bp product was amplified.

**Figure 5: RT-PCR to check for the expression of VPAC1 in B16F10 cells .**

Total RNA was isolated from the murine melanoma cell line B16F10. RT-PCR was carried out using gene specific primers for the amplification of the VPAC1 (SEQ ID3 and SEQ ID4).

**Figure 6: RT-PCR to check for the expression of VPAC-1 and Ub-VPAC-1 gene.**

The constructs pUb/VPAC and pcDNA were transfected in the murine melanoma cell line B16F10. 24 hours post- transfection, the total RNA was isolated from the transfected cells. The RNA samples were treated with DNAse1 to remove any contaminating plasmid contamination from the RNA sample. RT-PCR was carried out using gene- specific primers. **a** VPAC-1 expression was analysed by RT-PCR using primers corresponding to the SEQ ID 6 and SEQ ID4. **b** Ub-VPAC-1 gene was amplified using primers of SEQ ID 1 and SEQ ID4.

**Figure 7: In vitro CTL assays.**

Spleen cells of pUb-VPAC1 (Ub-VAC1), pVAC1 (VPAC1) and vector alone immunized mice were tested against B16F10 cells at different effector to target ratios using commercially available cytotox 96 non- radioactive cytotoxic assay kit.

**Figure 8: Abrogation of CTL mediated cytotoxicity by in vitro depletion of the immune cell subsets.**

Spleen cells of pUb-VPAC1 (Ub-VPAC1), pVAC1(VPAC1) and vector alone immunized mice were depleted of CD4+ or CD8+ lymphocytes as described in material and methods. The spleen cells were further tested against B16F10 cells at effector to target ratio of 50:1 using commercially available cytotox 96 non-radioactive cytotoxic assay kit.

**Induction of protective antitumor immunity**

*Tumor volume(mm³)* vs *Days after tumor injection*

Legend: vector — VPAC1 — Ub-VPAC1

**Figure 9: Induction of the protective antitumor immunity.**

Mice (9 mice in each group) were immunized I.m. with 100μg of pUbVPAC-1 (Ub-VPAC), pVPAC-1 (VPAC), or vector alone, once in 14 days three times. Mice were then challenged with $2 \times 10^6$ B16F10 cells s.c, after the third immunization. The figure represents the tumor volume (in mm³) of the different groups.

**Figure 10 : Induction of the therapeutic antitumor immunity.**

C57BL/6 mice( 10 in each group) implanted with B16F10 melanoma cells were treated with pUb-VPAC-1 (Ub-VPAC1), pVPAC-1(VPAC1) and vector alone. Anti tumor growth was evaluated by tumor growth.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Curr Opin Mol Ther,* 2001, vol. 3 (1), 77-84
- *Cancer Res,* 2002, vol. 62, 5807-5812
- *Curr Med Chem.,* 2006, vol. 13 (14), 1591-607
- *Cancer Res.,* 1998, vol. 58, 4902-4908
- *The Oncologists,* 2002, vol. 3, 20-33
- *Scand J Immunol.,* 2005, vol. 61, 391-397
- **Lewis Y ; Globo H ; GM2.** *Clin. Cancer Res.,* 1998, vol. 4, 295-302
- *Semin Oncol.,* 2003, vol. 30, 659-666
- *Mini Rev Med Chem.,* 2003, vol. 3, 633-649
- *Int J Cancer,* 1983, vol. 31, 567-575
- *Semin Sur. Oncol.,* 1989, vol. 5, 420-425
- *Cancer Res.,* 1992, vol. 52, 5948-5953
- *Semin Oncol.,* 2003, vol. 30 (5), 659-666
- *Cancer Res.,* 1991, vol. 51, 2731-2734
- *Cancer Res.,* 1999, vol. 59, 5160-5168
- *Immunol lett.,* 2000, vol. 74, 87-93
- *Prostate,* 1999, vol. 39, 54-59
- *Brit. Med.J.,* 2001, vol. 323, 1289-1293
- *J Clin Invest,* 2002, vol. 109, 409-417
- *Cytokines Cell Mol Ther.,* 1999, vol. 5, 139-144
- *Cytokines Cell Mol Ther,* 1999, vol. 5, 217-225
- *Semin Oncol.,* 2003, vol. 30, 659-666
- *Brit J Cancer,* 2005, vol. 93, 1085-1091
- *Annu Rev Immunol.,* 1997, vol. 15, 297-322
- *Immunity,* 1995, vol. 2, 271-279
- *Brit. Med J.,* 2001, vol. 323, 1289-1293
- *Expert Opin Biol Ther.,* 2006, vol. 6 (10), 967-970
- Handbook of Cancer Vaccines. 2004, 157-173
- *Vaccine,* 1994, vol. 12, 1503-1509
- *J Leukoc. Biol,* 2000, vol. 68, 793-806
- *Annu Rev. Immunol,* 2000, vol. 18, 927-974
- *Immunol. Cell Biol,* 1997, vol. 75, 376-381
- *Immunol Cell Biol.,* 1997, vol. 75, 370-375
- *Immunol. Today,* 1995, vol. 16, 338-342
- *Immunol Cell Biol.,* 1997, vol. 75, 364-369
- *Virology,* 1994, vol. 199, 132-140
- *Vaccine,* 1994, vol. 12, 1503-1509
- *Clin. Immuno.,* 1999, vol. 90, 100-107
- *DNA Cell Biol.,* 1993, vol. 12, 799-805
- *Blood,* 2003, vol. 102 (11), 746
- *Cellular Immunology,* 2002, vol. 215, 20-31
- *Annu. Rev. Biochem.,* 1993, vol. 62, 453-481
- *EMBO J.,* 1989, vol. 8, 2509-2517
- *Cell,* 1994, vol. 78, 335-342
- *Science,* 1989, vol. 243, 811-814
- *Current Science,* 2001, vol. 81 (5), 535-541
- *J. Immunol.,* 2003, vol. 170, 3162-3170
- *Pancreas,* 1998, vol. 17, 169-175
- *Clin. Cancer Res.,* 1999, vol. 5, 1063-1071
- *Nature Biotech.,* 2005, vol. 23, 1147-1157
- *J Immunol.,* 2003, vol. 170, 3162-3170
- *Cancer Res,* 2000, vol. 60, 3105-3112
- *N. Engl.J. Med.,* 1994, vol. 331, 1116-1121
- *PNAS,* 1993, vol. 90, 4345-4349
- *J. Mol Med,* 2002, vol. 80, 16-24
- *Cancer Res.,* 2000, vol. 60, 3105-311
- *Endocrine Rev.,* 2003, vol. 24 (4), 389-427
- *Endocrine Rev.,* 2003, vol. 24 (4), 389-427
- *FASEB J.,* 1997, vol. 11, 1245-1256
- *Annu. Rev. Biochem.,* 1998, vol. 67, 425-479
- *Bioessays,* 2000, vol. 22, 442-451
- *Nat. Rev. Mol. Cel. Biol,* 2001, vol. 2, 169-178
- *Science,* 1989, vol. 243, 1576-1583
- *Biochem. Biophyss. Res. Commun.,* 1999, vol. 266, 633-640
- *Nature,* 1993, vol. 363, 552-554
- *J.Cell Physiol.,* 2000, vol. 182, 1-11
- *J.Leuk Biol.,* 2000, vol. 68, 793-806
- *Mol. Therapy,* 2004, vol. 10 (3), 432-466
- *Gene therapy,* 2005, vol. 12, 1049-1057
- *Blood,* 2003, vol. 102 (13), 4535-4540
- Molecular Cloning--A laboratory Manual. Cold Spring Harbor Press, 2001

SEQUENCE LISTING

<110> DABUR PHARMA LIMITED
Maithal, Kapil
Sengupta, Paromita
Mukherjee, Rama

<120> DNA VACCINE FOR CANCER THERAPY

<130> DAB-3393-05

<160> 10

<170> PatentIn version 3.3

<210> 1

EP 1 962 891 B1

<211> 38
<212> DNA
<213> Artificial Sequence

<220>
<223> This oligonucleotide was artificially generated

<400> 1
aataagcttg ccaccatgca gattttcgtg aaaaccct        38

<210> 2
<211> 37
<212> DNA
<213> Artificial Sequence

<220> .
<223> This oligonucleotide was artificially generated

<400> 2
aatgatatca acaacaccac gaagtctcaa cacaaga        37

<210> 3
<211> 28
<212> DNA
<213> Artificial Sequence

<220>
<223> This oligonucleotide was artificially generated

<400> 3
aatgatatcg ccaggctgca ggaggagt        28

<210> 4
<211> 32
<212> DNA
<213> Artificial Sequence

<220>
<223> This oligonucleotide was artificially generated

<400> 4
aatctcgagt tagtagccgg tcttcacaga ac        32

<210> 5
<211> 37
<212> DNA
<213> Artificial Sequence

<220>
<223> This oligonucleotide was artificially generated

<400> 5
aataagcttg ccaccatggc caggctgcag gaggagt        37

<210> 6
<211> 21
<212> DNA
<213> Cytomegalo virus

<400> 6
cgcaaatggg cggtaggcgt g          21

<210> 7
<211> 228
<212> DNA
<213> Human

<400> 7

atgcagattt tcgtgaaaac ccttacgggg aagaccatca ccctcgaggt tgaaccctcg          60
gatacgatag aaaatgtaaa ggccaagatc caggataagg aaggaattcc tcctgatcag          120
cagagactga tctttgctgg caagcagctg gaagatggac gtactttgtc tgactacaat          180
attcaaaagg agtctactct tcatcttgtg ttgagacttc gtggtggt                      228

<210> 8
<211> 348
<212> DNA
<213> Human

<400> 8

gccaggctgc aggaggagtg tgactatgtg cagatgatcg aggtgcagca caagcagtgc          60
ctggaggagg cccagctgga gaatgagaca ataggctgca gcaagatgtg ggacaacctc          120
acctgctggc cagccacccc tcggggccag gtagttgtct tggcctgtcc cctcatcttc          180
aagctcttct cctccattca aggccgcaat gtaagccgca gctgcaccga cgaaggctgg          240
acgcacctgg agcctggccc gtaccccatt gcctgtggtt tggatgacaa ggcagcgagt          300
ttggatgagc agcagaccat gttctacggt tctgtgaaga ccggctac                      348

<210> 9
<211> 606
<212> DNA
<213> Human

<400> 9

aagcttgcca ccatgcagat tttcgtgaaa acccttacgg ggaagaccat caccctcgag          60
gttgaaccct cggatacgat agaaaatgta aaggccaaga tccaggataa ggaaggaatt          120
cctcctgatc agcagagact gatctttgct ggcaagcagc tggaagatgg acgtactttg          180
tctgactaca atattcaaaa ggagtctact cttcatcttg tgttgagact tcgtggtgtt          240
gttgatatcg ccaggctgca ggaggagtgt gactatgtgc agatgatcga ggtgcagcac          300
aagcagtgcc tggaggaggc ccagctggag aatgagacaa taggctgcag caagatgtgg          360

17

```
gacaacctca cctgctggcc agccacccct cggggccagg tagttgtctt ggcctgtccc    420

ctcatcttca agctcttctc ctccattcaa ggccgcaatg taagccgcag ctgcaccgac    480

gaaggctgga cgcacctgga gcctggcccg taccccattg cctgtggttt ggatgacaag    540

gcagcgagtt tggatgagca gcagaccatg ttctacggtt ctgtgaagac cggctactaa    600

ctcgag                                                              606
```

<210> 10
<211> 195
<212> PRT
<213> Artificial

<220>
<223> This fusion protein was artificially generated

<400> 10

```
Met Gln Ile Phe Val Lys Thr Leu Thr Gly Lys Thr Ile Thr Leu Glu
1               5                   10                  15

Val Glu Pro Ser Asp Thr Ile Glu Asn Val Lys Ala Lys Ile Gln Asp
            20                  25                  30

Lys Glu Gly Ile Pro Pro Asp Gln Gln Arg Leu Ile Phe Ala Gly Lys
            35                  40                  45

Gln Leu Glu Asp Gly Arg Thr Leu Ser Asp Tyr Asn Ile Gln Lys Glu
    50                  55                  60

Ser Thr Leu His Leu Val Leu Arg Leu Arg Gly Val Val Asp Ile Ala
65                  70                  75                  80

Arg Leu Gln Glu Glu Cys Asp Tyr Val Gln Met Ile Glu Val Gln His
                85                  90                  95

Lys Gln Cys Leu Glu Glu Ala Gln Leu Glu Asn Glu Thr Ile Gly Cys
            100                 105                 110

Ser Lys Met Trp Asp Asn Leu Thr Cys Trp Pro Ala Thr Pro Arg Gly
        115                 120                 125

Gln Val Val Val Leu Ala Cys Pro Leu Ile Phe Lys Leu Phe Ser Ser
    130                 135                 140

Ile Gln Gly Arg Asn Val Ser Arg Ser Cys Thr Asp Glu Gly Trp Thr
145                 150                 155                 160

His Leu Glu Pro Gly Pro Tyr Pro Ile Ala Cys Gly Leu Asp Asp Lys
                165                 170                 175

Ala Ala Ser Leu Asp Glu Gln Gln Thr Met Phe Tyr Gly Ser Val Lys
            180                 185                 190

Thr Gly Tyr
        195
```